(19) Europäisches Patentamt
European Patent Office
Office européen des brevets

(11) Numéro de publication: **0 038 758**
**B1**

(12) # FASCICULE DE BREVET EUROPEEN

(45) Date de publication du fascicule du brevet: **06.03.85**

(21) Numéro de dépôt: **81400621.9**

(22) Date de dépôt: **17.04.81**

(51) Int. Cl.[4]: **C 07 K 5/06**, C 07 C 149/00, C 07 C 101/26, C 07 C 103/44, C 07 C 125/065, C 07 C 153/07, A 61 K 31/195, A 61 K 31/21, A 61 K 37/02

(54) **Dérivés d'acides aminés et leur application thérapeutique.**

(30) Priorité: **17.04.80 FR 8008601**

(43) Date de publication de la demande:
**28.10.81 Bulletin 81/43**

(45) Mention de la délivrance du brevet:
**06.03.85 Bulletin 85/10**

(84) Etats contractants désignés:
**AT BE CH DE FR GB IT LI LU NL SE**

(56) Documents cités:
**DE-A-2 709 820**
**DE-A-2 720 996**
**DE-B-1 062 705**
**FR-A-2 201 100**
**FR-A-2 294 694**
**FR-A-2 372 804**
**FR-A-2 383 171**
**FR-A-2 383 917**
**FR-A-2 389 600**
**FR-A-2 414 498**
**FR-A-2 427 821**
**FR-M- 3 081**
**GB-A- 850 479**

(73) Titulaire: **SOCIETE CIVILE BIOPROJET**
**30, rue des Francs Bourgeois**
**F-75003 Paris (FR)**

(72) Inventeur: **Roques, Bernard**
**12 Rue Eugène Delacroix**
**F-94410 Saint-Maurice (FR)**
Inventeur: **Schwartz, Jean-Charles**
**9 Villa Seurat**
**F-75014 Paris (FR)**
Inventeur: **Lecomte, Jeanne-Marie**
**30 rue des Francs-Bourgeois**
**F-75003 Paris (FR)**

(74) Mandataire: **Bressand, Georges et al**
**c/o CABINET LAVOIX 2 Place d'Estienne d'Orves**
**F-75441 Paris Cedex 09 (FR)**

(56) Documents cités:
**GB-A-2 000 783**
**GB-A-2 054 586**
**US-A-3 246 025**
**US-A-3 700 770**
**US-A-4 112 119**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 038 758 B1**

CHEMICAL ABSTRACTS, vol. 92, no. 9, 3 mars 1980, page 724, abrégé 76902v, COLUMBUS OHIO (US)
CHEMICAL ABSTRACTS, vol. 91, no. 17, 22 octobre 1979, page 695, abrégé 141238z, COLUMBUS OHIO (US)
CHEMICAL ABSTRACTS, vol. 76, no. 3, 17 janvier 1972, page 417, abrégé 14875v, COLUMBUS OHIO (US)
CHEMICAL ABSTRACTS, vol. 77, no. 21, 20 novembre 1972, page 174, abrégé 136780b, COLUMBUS; OHIO (US)
CHEMICAL ABSTRACTS, vol. 78, no. 5, 5 février 1973, page 23, abrégé 23968w, COLUMBUS OHIO (US)
CHEMICAL ABSTRACTS, vol. 78, no. 3, 22 janvier 1973, page 337, abrégé 15445h, COLUMBUS OHIO (US)
CHEMICAL ABSTRACTS, vol. 81, no. 17, 28 octobre 1974, page 270, abrégé 102235v, COLUMBUS OHIO (US)

## Description

La présente invention est relative à de nouveaux dérivés d'amino-acides, à leurs procédés de préparation et leur application thérapeutique.

Les dérivés selon la présente invention sont des inhibiteurs de l'enképhalinase qui est une enzyme dégradant les enképhalines.

La méthionine-enképhaline, [Met-E (Tyr-gly-gly-Phe-Met), et la leucine enképhaline, Leu-E (Tyr-gly-gly-Phe-Leu) sont des peptides découverts dans le cerveau et qui sont les ligands endogènes du récepteur morphinique (J. Hughes et al. *Nature, 258*, 577 (1975)].

Leur action est multiple tant au niveau de la transmission des influx nociceptifs qu'au niveau du comportement ou de la sécrétion hormonale. Ils sont considérés comme des neuromédiateurs à action inhibitrice sur la libération d'autres neurotransmetteurs [J. Hughes, Nature, *278*, 394 (1979); S. H. Snyder, Nature, *278*, 13 (1979)]. Il a été démontré que les enképhalines sont rapidement dégradées dans le cerveau par une carboxydipeptidase qui libère les restes Tyr-gly-gly et Phe-Met (B. Malfroy et al, *Nature*, 276, 523 (1978).

Les composés susceptibles d'inhiber l'enképhalinase peuvent donc prolonger les effets des enképhalines endogènes ou potentialiser l'action d'analogues synthétiques administrés de façon exogène. Ces composés sont donc susceptibles de remplacer les agents morphiniques dans toutes leurs propriétés sans en avoir les graves inconvénients, en particulier au niveau des phénomènes d'accoutumance et de dépendance.

On connaît des thiopropanoylaminoacides ayant la structure partielle

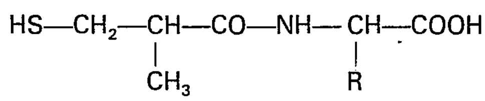

notamment d'après le brevet US—A—4 112 119 et les demandes FR—A—2 383 171 et FR—A—2 372 804. Ces composés ont des propriétés inhibitrices de l'angiotensine-convertase (ACE) et n'ont pratiquement aucune activité sur l'enképhaline, en particulier à cause du groupe $CH_3$ présente sur la chaîne latérale (ou un groupement équivalent).

Or, la présente invention vise des composés agissant sur l'enképhalinase mais pas sur l'ACE, ces deux enzymes étant bien distinctes et ayant des fonctions biologiques totalement différentes.

Les dérivés selon l'invention répondent à la formule générale

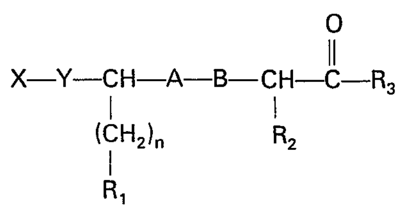 (I)

dans laquelle;

*A* est un groupe carbonyle, méthylène ou amino;

*B* est un groupe amino, carbonyle ou thio;

$R_1$ est un groupe phényle éventuellement mono- ou polysubstitué par un atome d'halogène,

*n* est 0 ou 1;

$R_2$ est un atome d'hydrogène, un groupe alkyle en $C_1$ à $C_6$ linéaire ou ramifié; un groupe benzyle; un groupe benzyloxyalkyle.

$R_3$ est un groupe $OR_4$, $NHR_4$ ou $N(R_4)_2$, dans lesquels $R_4$ est un atome d'hydrogène; un groupe alkyle linéaire ou ramifié en $C_1$—$C_6$ éventuellement mono- ou polysubstitué par un atome d'halogène; un groupe phényle ou phénylalkyle ($C_1$—$C_4$), ces deux derniers groupes étant éventuellement mono- ou polysubstitués sur le noyau phényle par un atome d'halogène; un groupe dialkyl aminoalkyle, les groupes alkyle étant en $C_1$ à $C_4$ et le groupe amino pouvant être sous forme aminoxy;

*Y* est un groupe —S—; —NH—; —$CH_2$—; ou bien *Y* est un groupe amino tertiaire dont la troisième valence forme avec $R_1$ un point alcoylène à 2 atomes de carbone; et

*X* est un atome d'hydrogène; un groupe alkyle en $C_1$ ou $C_2$ substitué par un radical alcoxycarbonyle, carboxy, mercapto, un radical acyle aliphatique ($C_{1-4}$) thio ou benzoylthio; un groupe acyle aliphatique en $C_1$ à $C_4$ ou benzoyle; un groupe mercapto; un groupe acyle aliphatique ($C_{1-4}$) thio; un groupe benzoylthio ou un groupe phénylalkyl ($C_{1-4}$) carbonylthio éventuellement mono- ou polysubstitué par un atome d'halogène; un groupe hydroxyalkyl ($C_{1-4}$) carbonylthio ou aminoalkyl ($C_{1-4}$) carbonylthio;

et leurs sels d'addition avec des acides ou des bases pharmaceutiquement acceptables, sous réserve que l'on n'ait pas simultanément $X=R_2=H$, $Y=CH_2$ et n=0 et, de plus, sous réserve que dans le cas A=CO et B=NH, lorsque Y=—S—, X soit différent de H et de benzoyle et lorsque Y=—$CH_2$—, X

soit différent du groupe alkyle au $C_1$ ou $C_2$ substitué par un radical alcoxy carboxyle ou carboxy, ou $R_2$ ne représente pas un groupe alkyle en $C_2$—$C_5$.

Parmi les composés de formule I définis ci-dessus, une classe préférée de composés comprend les dérivés dans lesquels:

*A* est un groupe carbonyle, méthylène ou amino;

*B* est un groupe amino, carbonyle ou thio;

$R_1$ est un groupe phényle éventuellement mono- ou polysubstitué par un atome d'halogène;

*n* est 0 ou 1;

$R_2$ est un atome d'hydrogène; un groupe alkyle en $C_1$ à $C_6$ linéaire ou ramifié; un groupe benzyle; un groupe benzyloxyalkyle;

$R_3$ est un groupe $OR_4$ ou —$NHR_4$, dans lequels $R_4$ est un atome d'hydrogène; un groupe alkyle linéaire ou ramifié en $C_1$ à $C_6$, éventuellement mono- ou polysubstitué par un atome d'halogène; phénylalkyle ($C_{1-4}$) éventuellement mono- ou polysubstitué sur le noyau phényle par un atome d'halogène;

*Y* est un groupe —S—; —NH—, —$CH_2$—, ou bien Y est un groupe amino tertiaire dont la troisième valence forme avec $R_1$ un pont alcoylène à 2 atomes de carbone; et

*X* est un atome d'hydrogène; un groupe alkyle en $C_1$ ou $C_2$ substitué par un radical alcoxycarbonyle, carboxy, mercapto, acyle aliphatique ($C_{1-4}$) thio, ou benzoylthio; un groupe acyle aliphatique en $C_1$ à $C_4$ ou benzoyle; un groupe mercapto; un groupe acyle aliphatique ($C_{1-4}$) thio; un groupe benzoylthio ou un groupe phénylalkyl ($C_{1-4}$) carbonylthio.

A titre d'atome d'halogène, on préfère particulièrement de fluor. Comme groupe acyle aliphatique on peut mentionner les groupes acétyle, propionyle et butyryle, le groupe acétyle étant préféré.

On préfère, parmi les composés de formule I, ceux qui décrivent les structures amino-acides suivantes: leucine, phénylalanine, glycine, sérine et alanine.

L'invention envisage, à titre de composés spécifiques, les dérivés suivants (dans les formules données, on désigne par Et un radical éthyle et par $\phi$ un groupe phényle):

1) $EtO_2C$—$CH_2$—L—Phe—L—Leu—$OCH_3$

2) $HO_2C$—$CH_2$—L—Phe—L—Leu OH

3) $HO_2C$—$CH_2$—L—Phe—($CH_3$)—L—Leu OH

4) $HO_2C$—$CH_2$—L—Phe—D Leu OH

5) $HO_2C$—$CH_2$—D—Phe—L—Leu OH

6) $HO_2C$—$CH_2$—D—Phe—D—Leu OH

7) $EtO_2C$—$CH_2$—$CH_2$—L—Phe—L—Leu $OCH_3$

8) $HO_2C$—$CH_2$—$CH_2$—L—Phe—L—Leu OH

9) $EtO_2C$—$CH_2$—L—Phe—L. Ala—$OCH_3$

10) $HO_2C$—$CH_2$—L—Phe—L. Ala—OH

11) HS—$CH_2$—$CH_2$—L—Phe Otbu

12) $\phi$—COS—$CH_2$—$CH_2$—L. Phe Otbu

13) $\phi$—COS—$CH_2$—$CH_2$—L. Phe OH

14) $\phi$—COS—$CH_2$—$CH_2$—L—Phe—L—Leu $OCH_3$

15) HS—$CH_2$—$CH_2$—L—Phe—L—Leu OH

16) HS—$CH_2$—$CH_2$—L—Phe—L—Ala OH

17) $CH_3$—COS—$CH_2$—CH(—$CH_2$—$\phi$)—CO—L—Leu $OCH_3$ (R, S et S, S)

18) 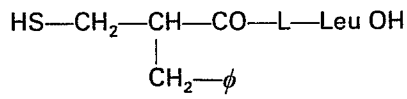 (R, S et S, S)

19) 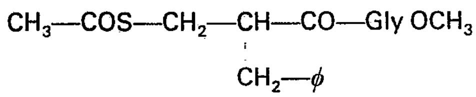 (R et S)

20) 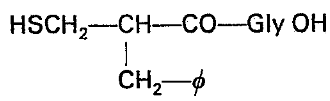 (R et S)

21) 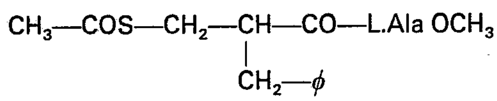 (R, S et S, S)

22) 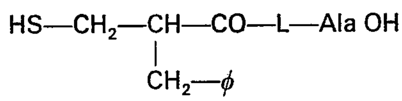 (R, S, et S, S)

23) 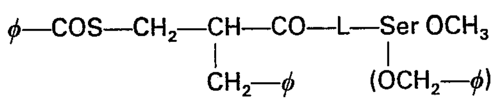 (R, S et S, S)

24) 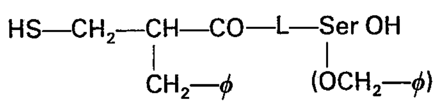 (R, S et S, S)

25) 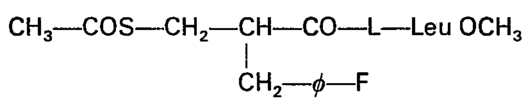 (R, S et S, S)

26) 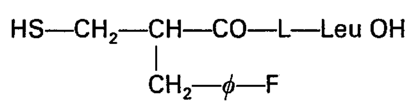 (R, S et S, S)

27) 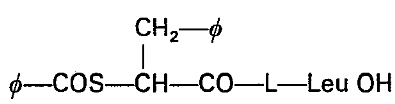 (S, S)

28) 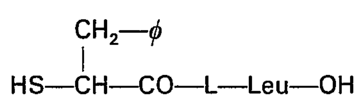 (S, S)

29) 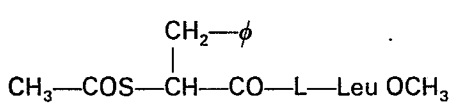 (S, S)

30)

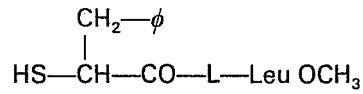

(S, S)

31)

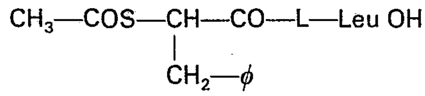

(S, S)

32)

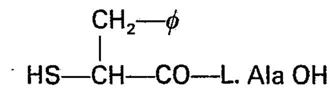

(S, S)

33)

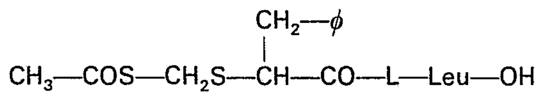

(S, S)

34)

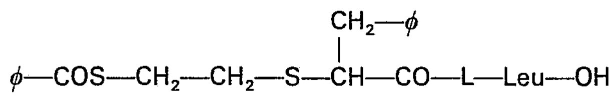

(S, S)

35)

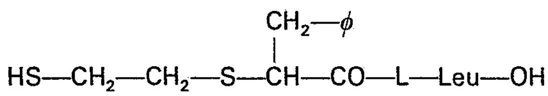

(S, S)

36)

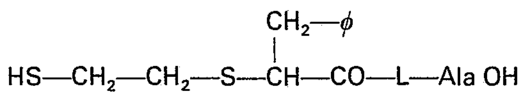

(S, S)

37)

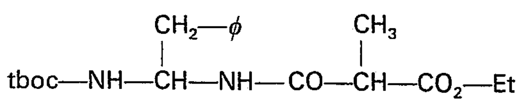

(SR+SS)

38)

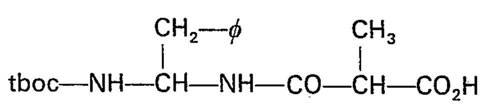

(SR+SS)

39)

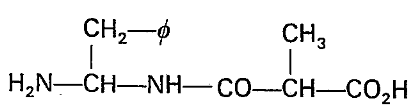

(RR+RS)

40)

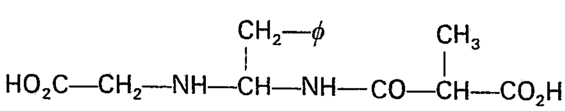

(RR+RS)

41)

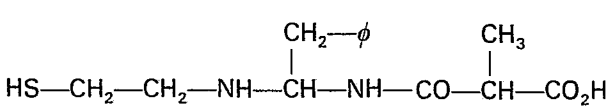

(RR+RS)

42)

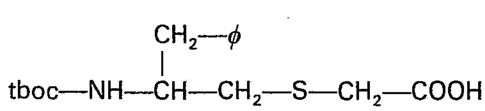

(S)

43) 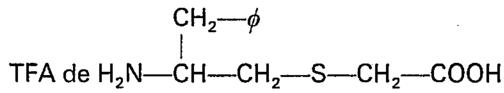 (S)

44) 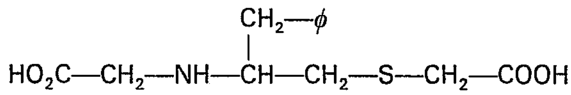 (S)

45) 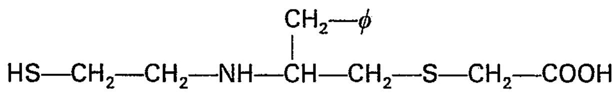 (S)

46) 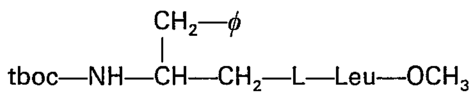 (S, S)

47) 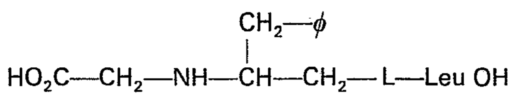 (S, S)

48) 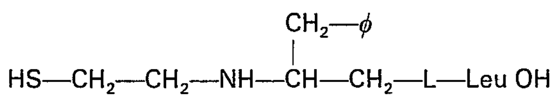 (S, S)

49) $EtOCOCH_2—L—Pro—L—Ala\ OCH_3$

50) $HOOC—CH_2—L—Pro—L—Ala\ OH$

51) 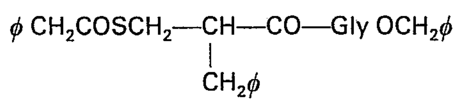 (R, S)

52) 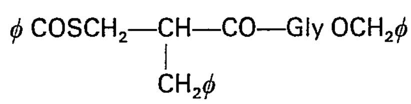 (R, S)

53) 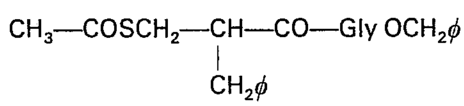 (R, S)

54) 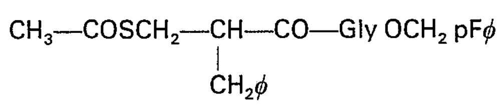 (R, S)

55) 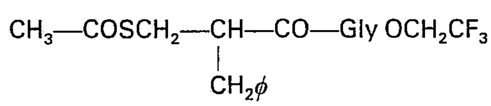 (R, S)

56) 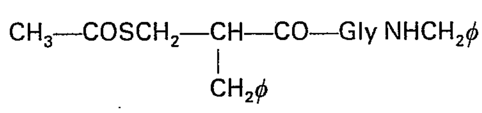 (R, S)

57)

$HSCH_2CH—CO—Gly\ OCH_2\phi$ (R, S)
|
$CH_2\phi$

58)

$HSCH_2—CH—CO—Gly\ OCH_2\ pF\phi$ (R, S)
|
$CH_2\phi$

59)

$HSCH_2—CH—CO—Gly\ OCH_2\ CF_3$ (R, S)
|
$CH_2\phi$

60)

$HSCH_2CH—CO—Gly\ NHCH_2\phi$ (R, S)
|
$CH_2\phi$

Les composés de formule I ont un ou deux atomes de carbone asymétriques. Ils existent donc sous forme de mélange racémique ou sous des formes diastéréoisomères. Tous ces composés entrent dans le cadre de la présente invention. Les synthèses décrites ci-après peuvent utiliser le racémique ou l'un des énantiomères comme substance de départ. Quand on utilise dans le mode opératoire de synthèse la substance de départ racémique, on peut séparer les stéréoisomères obtenus dans le produit par des procédés classiques de chromatographie ou de cristallisation fractionnée. En général, l'isomère L par rapport à l'atome de carbone de l'amino-acide constitue la forme isomère préférée.

Les composés de formule I forment des sels qui font également partie de cette invention. Les sels comprennent les sels d'addition d'acide que l'on forme par réaction avec divers acides minéraux et organiques fournissant des sels d'addition d'acide, comprenant, par exemple, les halohydrates (en particulier le chlorhydrate et le bromhydrate), le sulfate, le nitrate, le borate, le phosphate, l'oxalate, le tartrate, le maléate, le citrate, l'acétate, l'ascorbate, le succinate, le benzènesulfonate, le méthanesulfonate, le cyclohexane sulfonate et le toluènesulfonate.

On forme les sels d'addition de base par réaction avec des bases telles que NaOH ou par réaction d'échange d'ions.

On forme les sels d'une manière classique en faisant réagir la forme libre du produit avec un ou plusieurs équivalents de la base ou de l'acide approprié fournissant l'anion ou le cation désiré dans un solvant ou un milieu dans lequel le sel est insoluble ou dans l'eau et en éliminant l'eau par lyophilisation. En neutralisant le sel par un acide insoluble comme une résine échangeuse de cations sous forme hydrogène [par exemple la résine polystyrène-acide sulfonique-Dowex 50 (Mikes, Laboratory Handbook of Chromatographic Methods (Van Nostrand, 1961) page 256], an éluant avec un tampon volatil (par exemple pyridine/acide acétique) et en extrayant avec un solvant organique, on peut obtenir la formule libre et si on le désire on peut former un autre sel.

Les composés de la présente invention sont préparés par les divers procédés définis ci-après.

Les composés de formule I peuvent être préparés par une réaction de condensation peptidique classique entre deux restes acides aminés convenablement protégés.

Par exemple, le ou les groupes fonctionnels (c'est-à-dire les groupes amino, carboxy, hydroxy) qui ne sont pas impliqués dans la réaction de formation de la liaison peptide (c'est-à-dire —CONH) lors de la réaction de condensation peuvent être protégés par un ou des groupes protecteurs avant la réaction de condensation.

Comme groupes protecteurs intermédiaires des groupes amino, on emploie des groupes usuels tels que t.butoxycarbonyle (Boc), benzyloxycarbonyle (Z), isobornyloxycarbonyle (IBOC), etc.

Les groupes carboxyliques peuvent également être protégés, si nécessaire, par estérification (par exemple esters méthyliques, éthyliques, benzyliques, etc.).

On réalise la réaction de condensation en utilisant de préférence le couplage des azotures sans racémisation ou le procédé au dicyclohexyl carbodiimide/1-hydroxy-benzotriazole dit ci-après DCC/HOBT ou DCC/3-hydroxy-4-oxo-3,4-dihydro-1,2,3-benzotriazine (OOBt). On peut utiliser en variante les esters activés des fragments.

La réaction de condensation peut être effectuée en présence d'un solvant. Le solvant peut être choisi parmi ceux qui sont connus pour être utilisables dans les réactions de condensation de peptides. Ainsi, on peut mentionner à titre d'exemples les solvants anhydres ou aqueux ci-après: diméthylformamide, diméthylsulfoxyde, pyridine, chloroforme, dioxane, dichlorométhane, tétrahydrofurane, ainsi que des mélanges appropriés de tels solvants.

La température réactionnelle est choisie dans la gamme connue pour les réactions conduisant à la formation des liaisons peptides, par exemple normalement dans la gamme d'environ −20°C à environ

30°C. De plus, les matières précurseurs (peptides protégés) des composés recherchés selon la présente invention peuvent également être facilement préparés par des procédés de synthèse en phase solide.

Après la fin de la réaction de condensation recherchée, si le produit porte des groupes protecteurs, ils peuvent être éliminés par des procédés habituels. Parmi de tels procédés habituels, on peut citer: la réduction catalytique en présence d'un catalyseur tel que le noir de palladium, le carbone sur palladium, le platine, etc., la solvolyse au moyen d'acide fluorhydrique, d'acide trifluoroacétique, etc. et la réduction avec le sodium métallique dans l'ammoniac liquide.

On utilise notamment l'acide trifluoroacétique (TFA) pour éliminer les groupes Boc (amino protecteurs) et une saponification pour éliminer les groupes esters protecteurs des groupes carboxyliques.

On peut également préparer les composés de formule I par condensation entre une fraction d'un dérivé halogéné et la fraction correspondante d'un dérivé amino ou mercapto.

Selon un premier mode de réalisation, les composés de formule I dans lesquels A=CO, B=NH, Y=NH, X est un groupe alkylène en $C_1$ ou $C_2$ substitué par un groupe alcoxycarbonyle ou carboxy et $R_1$, $R_2$, $R_3$ et *n* sont tels que définis ci-dessus, sont préparés par réaction d'un dérivé de formule

$$ROOC—(CH_2)_mBr \qquad (II)$$

dans laquelle R est H ou un groupe alkyle et *m* est 1 ou 2, avec un dérivé de formule

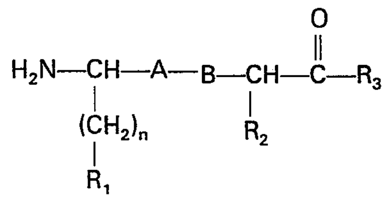

(III)

dans laquelle $R_1$, $R_2$ et $R_3$ sont tels que définis ci-dessus et A=CO; B=NH.

Les composés de formule III sont obtenus par les voies classiques de la synthèse peptidique, c'est-à-dire par couplage d'un dérivé protégé

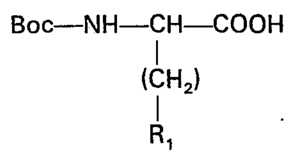

avec l'ester ou l'amide

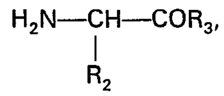

par utilisation des différentes méthodes connues précédemment indiquées telles que HOBT/DCC, anhydride mixte, ester activé. Le groupe protecteur Boc est ensuite éliminé par exemple à l'aide d'acide trifluoroacétique (TFA).

Selon un autre mode de réalisation, les composés de formule I dans lesquels A=$CH_2$, B=NH, Y=S, X est un atome d'hydrogène ou un groupe benzoyle, $R_1$ est H, *n*=O, $R_2$ et $R_3$ sont tels que définis ci-dessus, sont préparés

a) par réaction de thiiranne de formule $C_2H_4S$ sur un ester ou amide d'amino-acide de formule

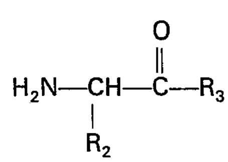

(III)

et

b) éventuellement réaction du produit obtenu à l'étape (a) avec un composé de formule

$$R—CO—Br$$

dans laquelle R est un groupe alkyle inférieur ou phényle et élimination des groupes protecteurs.

Selon un autre mode de réalisation de la présente invention, les composés de formule I dans lesquels A=CO, B=NH, Y=NH ou $CH_2$, X est un groupe alkyle en $C_1$ ou $C_2$ substitué par un groupe

mercapto, acyle aliphatique thio ou benzoylthio, $R_1$, $R_2$, $R_3$ et *n* sont tels que définis ci-dessus, sont préparés par une réaction de condensation peptidique entre un composé de formule

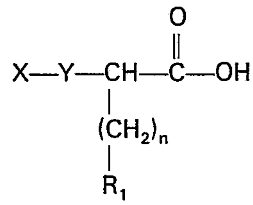

(IV)

et
un ester ou un amide d'amino-acide de formule

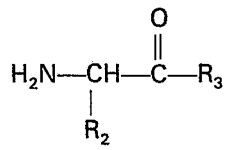

Lorsque le composé de formule IV comporte un groupe Y qui est un groupe —NH—, on le prépare comme au mode de réalisation précédent.

Lorsque le composé de formule IV comporte un groupe Y qui est un groupe —$CH_2$—, il est obtenu par réaction d'un S-acide thiocarboxylique de formule R—COSH, dans laquelle R est un radical alkyle, phényle ou benzyle éventuellement mono- ou polysubstitué par un atome d'halogène, sur un dérivé d'acide acrylique de formule

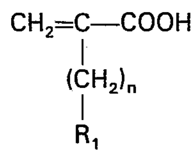

préparé selon C. Mannich et K. Ritsert, 1924, Ber *57*; 116), pour obtenir un composé de formule

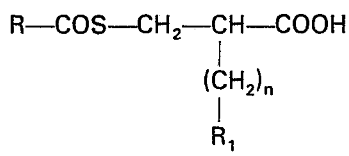

(IVa)

Selon un autre mode de réalisation de la présente invention, les composés de formule I dans lesquels A=CO, B=NH, Y=S, X est, un groupe acyle aliphatique en $C_1$ à $C_4$ et $R_1$, $R_2$, $R_3$ et *n* sont tels que définis ci-dessus, sont préparés par réaction d'un S-acide thiocarboxylique de formule R—COSH, dans laquelle R est un radical alkyle sur un dérivé halogéné de formule

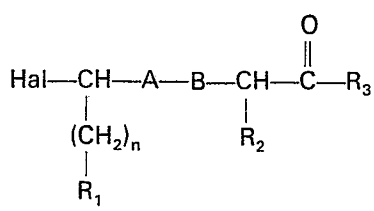

(V)

dans laquelle Hal est un atome d'halogène en particulier de brome.

Les composés de formule (V) sont obtenus par une réaction de couplage analogue à la synthèse peptidique entre un bromo-acide de formule

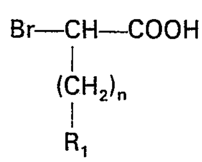

et un ester ou amide d'amino-acide de formule

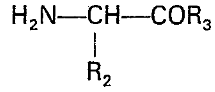

Selon une variante de ce mode de réalisation il est possible de faire tout d'abord réagir le bromo-acide avec le S-thioacide carboxylique, puis de coupler l'acide obtenu avec l'ester ou l'amide d'amino-acide.

Selon encore un autre mode de réalisation de la présente invention, les composés de formule I dans lesquels A=CO, B=NH, Y=S, X est un groupe alkyle en $C_1$ ou $C_2$ substitué par un groupe mercapto, acyle aliphatique thio ou benzoylthio et $R_1$, $R_2$, $R_3$ et *n* sont tels que définis ci-dessus, sont préparés par réaction d'un composé halogéné de formule $R—CO—S—(CH_2)_m—Hal$, dans laquelle Hal est un atome d'halogène tel que le brome, R est un groupe alkyle ou phényle et *m* est 1 ou 2, avec un dérivé de formule

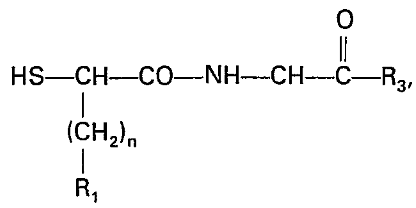

ce dernier composé étant obtenu à partir d'un composé de formule I dont la synthèse a été précédemment décrite, par libération du groupe mercapto terminal.

La synthèse des composés de formule I dans lesquels $Y=CH_2$ et X est un groupe acyle aliphatique thio; un groupe phénylalkyl $(C_{1-4})$ carbomylthio dans lequel le reste aromatique est mono- ou polysubstitué par un atome d'halogène; un groupe hydroxyalkylcarbonylthio ou un groupe aminoalkyl carbonylthio, est réalisée par condensation des chlorures d'acides correspondants en présence du mélange DCC+HOBT sur les dérivés I dans lesquels $Y=CH_2$ et X=SH.

L'obtention des dérivés de formule I à terminaison mercapto est obtenue par saponification à l'aide de soude des dérivés à terminaison acyle (aliphatique ou aromatique) thio. Cette saponification conduit à une prépondérance des composés de duplication à liaison disulfure, cette réaction d'oxydation s'effectuant majoritairement même en employant des solvants dégazés et en opérant sous atmosphère protectrice d'azote. Les composés de formule (I) à terminaison mercapto sont donc obtenus purs en effectuant une réduction in situ à l'hydrogène naissant au terme de la réaction de saponification.

Les composés I dans lesquels $Y=CH_2$ et X=SH et $R_1$, $R_2$ et $R_3$ sont tels que définis ci-dessussont préparés par la suite de réactions.

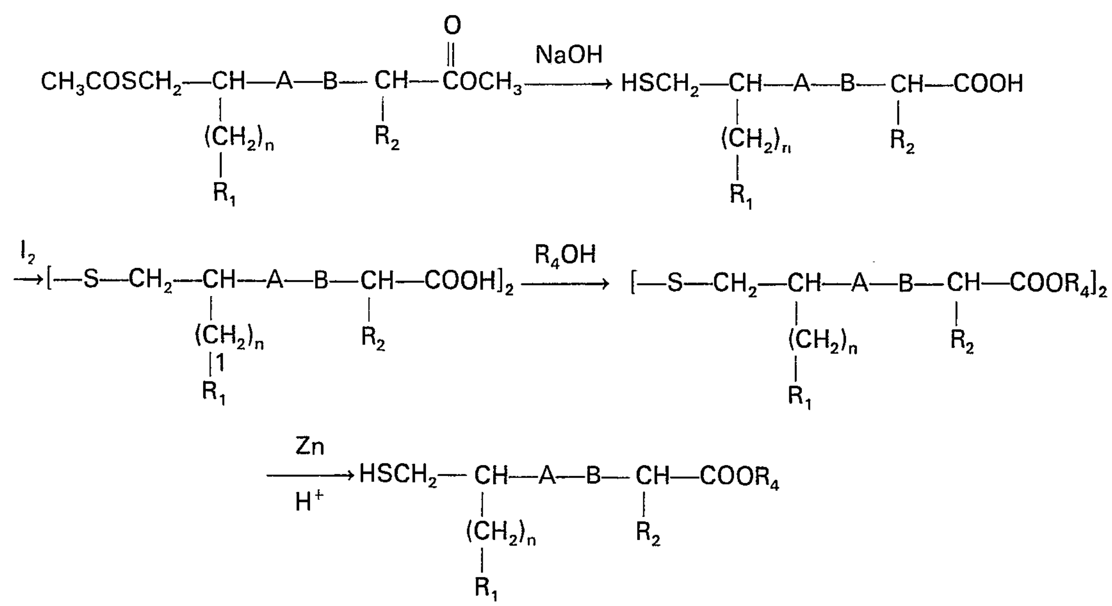


Lorsque dans la formule générale (I) le reste $R_3$ est un groupe amine primaire ou secondaire, la saponification directe du dérivé acétylthio permet d'obtenir directement les dérivés I avec X=SH et $Y=CH_2$.

Selon un autre mode de réalisation, les composés de formule I dans lesquels A=NH, B=CO,

Y=NH, X est un atome d'hydrogène, un groupe alkyle substitué par un groupe carboxy ou mercapto, ou un groupe alcoxycarbonyle et $R_1$, $R_2$ et $R_3$ sont tels que définis ci-dessus, sont préparés par une réaction de condensation peptidique entre une diamine N-protégée de formule

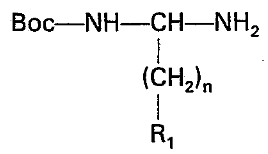

(VI)

et
un monoester ou monoamide malonique de formule

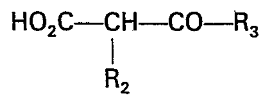

La diamine de formule (VI) est obtenue par une réaction de Curtius selon M. Chorev et al. [J. Amer. Chem. Soc., *99*, 8075 (1977)] sur un amino-acide N-protégé selon le schéma réactionnel suivant

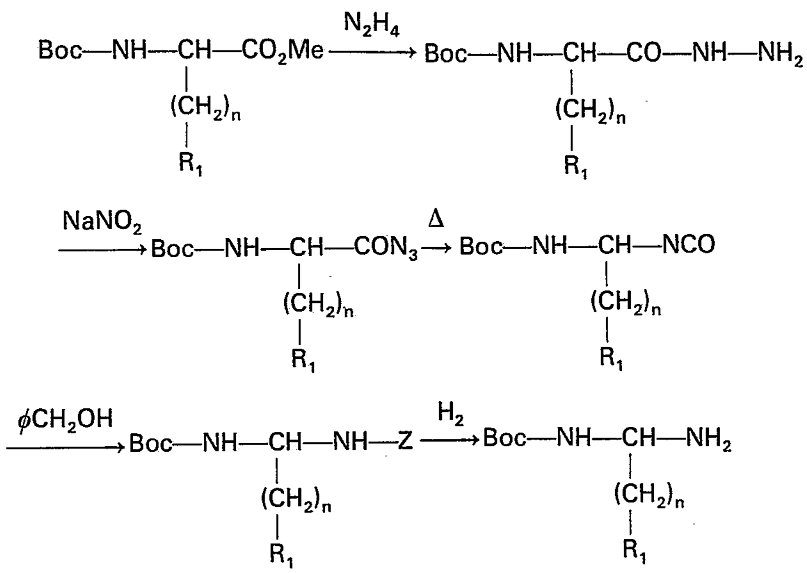


Selon un autre mode de réalisation, les composés de formule I dans lesquels $A=CH_2$, B=S, Y=NH, X est un atome d'hydrogène, un groupe alkyle substitué par un groupe carboxy ou mercapto, ou un groupe alcoxycarbonyle et $R_1$, $R_2$, et $R_3$ sont tels que définis ci-dessus, sont préparés par une réaction de condensation entre un dérivé toslyé N-protégé d'amino-acide de formule

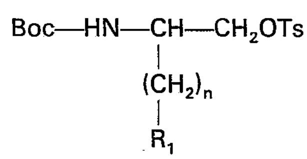

(VII)

et
un ester ou amide de l'acide thioglycolique de formule

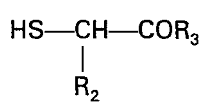

(VIII),

d'après la méthode de H. Gilman et al., J. Amer. Chem. Soc. *47*, 1449 (1925).

Les composés de formule VII sont obtenus par réduction au borohydrure de sodium (selon la méthode de H. Seki et al., Chem. Parm. Bull. 13, 995—VII—1965) d'un ester N-protégé d'amino-acide de formule

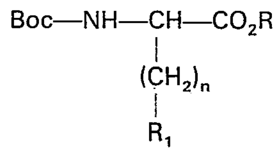

dans lequel R est un groupe alkyle, puis transformation de l'alcool obtenu en dérivé tosylé de formule VII par action de l'acide p-toluènesulfonique.

Les composés de formule VIII sont obtenus par la méthode de E. Fischer et al. Annalen. *357*, 1 (1907) ou décrite dans la DOS 2 349 707 selon le schéma réactionnel suivant

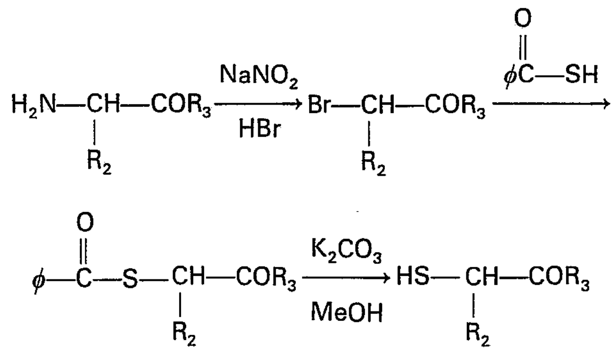


Selon un autre mode de réalisation, les composés de formule I dans lesquels A=—$CH_2$—, B=—NH—, Y=NH, X est un atome d'hydrogène, un groupe alkyle substitué par un groupe carboxy ou mercapto, ou un groupe alcoxycarbonyle et $R_1$, $R_2$ et $R_3$ sont tels que définis ci-dessus, sont préparés par une réaction de condensation entre un dérivé tosylé N-protégé d'amino-acide de formule VII (décrit ci-dessus) et un ester ou amide d'amino-acide de formule

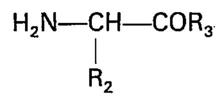

d'après la technique de V. C. Sekera et al., J. Amer. Chem. Soc. *55*, 345 (1933).

Les exemples ci-après sont donnés à titre d'illustration des procédés de préparation des composés selon la présente invention.

Exemple 1

Ester méthylique de la N(éthoxycarbonyl-méthyl)-L. phénylalanine-L. leucine

On ajoute lentement à froid et sous azote 3 ml de triéthylamine à une suspension de 10 g de trifluoroacétate de dipeptide-ester: L. Phe-L. Leu $OCH_3$ dans 50 ml de benzène anhydre. A la fin de l'addition, la suspension devient limpide. On ajoute alors 3,34 g de bromoacétate d'éthyle et la solution est portée au reflux pendant 12 heures. Après refroidissement, on ajoute goutte à goutte, sur un bain de glace, de la soude 0,25 N, jusqu'à pH=6.

La phase aqueuse est rapidement décantée, puis extraite trois fois (25 ml) à l'éther. Les phases organiques (éther et benzène) sont réunies, lavées deux fois avec 50 ml d'eau, puis séchées et évaporées à sec. On obtient 2,6 g de N-(éthoxycarbonyl-méthyl)-L. Phe.-L. Leu $OCH_3$ sous forme d'huile jaune pâle. Rf=0,77 dans $CHCl_3$/MeOH/$H_2O$/9/1/Sat.

Exemple 2

N-(carboxy-méthyl) L. phénylalanine-L. leucine

2,5 g de l'ester méthylique du N-(éthoxycarbonyl-méthyl)-L. Phe. L. Leu sont mis en solution dans un mélange de 20 ml de méthanol et 10 ml d'eau. On ajoute à 0°C, 13 ml de NaOH 1N et on agite 1 heure à 0°C, puis 2 heures à 25°C. On évapore le méthanol et additionne HCl 1N, goutte à goutte, à 0°C, jusqu'à pH=2. Le précipité est filtré et recristallisé dans le mélange éther/MeOH: 50/50; on obtient 1,35 g de N(carboxyméthyl)-L. Phe-L. Leu. F=196—200°C. Rf: (BuOH/AcOH/$H_2O$: 4/1/1)=0,6.

Exemple 3

N-(carboxyméthyl) L. phénylalanine-N ($CH_3$) L. leucine

En remplaçant l'ester méthylique de la L. leucine par l'ester méthylique de la N($CH_3$) L. leucine dans le procédé décrit à l'exemple 1, puis en traitant le produit obtenu selon l'exemple 2, on obtient successivement, l'ester méthylique du N(éthoxycarbonyl-méthyl)-L. Phe-N ($CH_3$) L. Leu, puis le N (carboxy-méthyl)-L. Phe-N ($CH_3$) L. Leu.

Lors de cette synthèse le composé de départ le TFA de L. Phe-N ($CH_3$) L. Leu $OCH_3$ est obtenu par

condensation en présence de DCC et HOBt de la Boc Phe avec l'ester méthylique de la N($CH_3$) L. Leu, suivie de déprotection par le TFA.

Exemple 4
N-(carboxy-méthyl)-L. phénylalanine-D. leucine

Obtenue comme dans l'exemple 1 en substituant la D. leucine à la forme L dans le composé de départ.

On obtient successivement l'ester méthylique du N-(éthoxycarbonyl-méthyl)-L-Phe-D. leu, puis le (carboxy-méthyl)-L-Phe-D. Leu. F=240°C.

Exemple 5
N-(carboxy-méthyl)-D. phénylalanine-L-leucine

Obtenue comme dans l'exemple 1 en substituant la D. phénylalanine à la forme L dans le composé de départ. On obtient successivement l'ester méthylique de la N-(éthoxycarbonyl méthyl)-D. Phe-L. Leu, puis le N-(carboxy-méthyl)-D-Phe-L-Leu. F=234°C.

Exemple 6
N-(carboxyméthyl)-D. phénylalanine-D. leucine

Obtenue comme dans l'exemple 1 en remplaçant la L. leucine et la L. phénylalanine par leurs isomères D. On obtient successivement l'ester méthylique de la N-(éthoxycarbonyl-méthyl)-D-Phe-D. Leu, puis la N-[(carboxy) méthyl] D. Phe-D-Leu. F=227°C.

Exemple 7
Ester méthylique du N-(éthoxycarbonyl-2 éthyl) L. Phe-L. Leu

8,80 g de Phe-Leu $OCH_3$ (obtenu sous forme de base à partir du TFA de Phe-Leu $OCH_3$ par action de la triéthylamine) sont mis en solution dans 200 ml de benzène anhydre sous atmosphère d'azote. On ajoute goutte à goutte à 0°C, 5,8 ml de bromopropionate d'éthyle et 2,07 g de $K_2CO_3$. On chauffe à 80°C durant 4 heures. Après refroidissement, la phase benzénique est rapidement séparée. On ajoute alors 20 ml d'$H_2O$ et goutte à goutte HCl 1N jusqu'à pH=2.

La phase benzénique est séparée, puis la phase aqueuse acide est extraite trois fois avec 50 ml d'acétate d'éthyle. Les phases organiques réunies sont séchées, puis évaporées à sec. On obtient 2,94 g d'huile jaune paille, qui est purifiée par chromatographie sur une colonne de gel de silice avec un mélange $Et_2O$/MeOH, 9/1 comme éluant. On obtient 1,10 g de l'ester méthylique de N(éthoxycarbonyl-2 éthyl) L-Phe-L. Leu, huile incolore. Rf=0,81, $CHCl_3$/MeOH/$H_2O$, 0/1/Sat.

Exemple 8
N-(carboxy-2 éthyl) L. Phe-L. Leu

On introduit 720 mg de l'ester méthylique du N-(éthoxycarbonyl-2 éthyl) L-Phe-L-Leu dans 10 ml de MeOH. On ajoute à 0°C, 7,2 ml de NaOH 1N. On agite 12 heures à température ordinaire. On acidifie par HCl jusqu'à pH 3 et on extrait la phase aqueuse avec trois fois 20 ml d'acétate d'éthyle. Les phase organiques séchées et évaporées donnent 510 mg de N(carboxy-2 éthyl) L-Phe-L. Leu sous forme de solide blanc. F=161°C. Rf=0,51. BuOH/AcOH/$H_2O$.

Exemple 9
Ester méthylique de N-(éthoxycarbonyl-méthyl) L. Phe-L. Ala.

Ce composé est obtenu comme à l'exemple 1 en remplaçant le TFA de L-Phe-L-Leu $OCH_3$ par le TFA de L. Phe-L. Ala $OCH_3$ (5 g). On obtient 1,10 g de l'ester méthylique du N-(éthoxycarbonyl-méthyl) L. Phe-L. Ala sous forme d'huile. Rf=0,71 dans $CHCl_3$/MeOH/$H_2O$,9/1/Sat.

Exemple 10
N-(carboxy-méthyl) L. Phe-L. Ala

La réaction est effectuée comme à l'exemple 2 en utilisant l'ester méthylique du N-(éthoxy-carbonyl-méthyl) L. Phe-L. Ala (1 g). On obtient après saponification et acidification 88 mg de N-(carboxy-méthyl) L-Phe-L. Ala. Rf=0,37 BuOH/MeOH/$H_2O$ 4/1/1.

Exemple 11
Ester t-butylique de la N-(mercapto-2 éthyl) L. Phe

On ajoute en ampoule 0,9 ml de thiiranne $C_2H_4S$, fraîchement distillé, à 8,7 g de L. Phe OtBu huileux obtenu sous forme de base à partir de son chlorhydrate. Le tube est scellé puis chauffé 5 heures à 100°C. Après refroidissement, le tube est ouvert et le liquide sirupeux est distillé.

On obtient 3,62 g de l'ester t. butylique de la N(mercapto-2 éthyl) L. Phe. $Eb_{0,5}$=130—134°C.

Exemple 12
Ester t-butylique de la N(benzoyl thio-2 éthyl)-L-Phe

3,62 g de l'ester t-butylique du N(mercapto-2 éthyl) L-Phe, sont mis en solution dans 15 ml de

$H_2O$ additionné de 628 mg de $K_2CO_3$. On ajoute lentement 1,51 ml de bromure de benzoyle dissous dans 15 ml d'éther. On agite 1 heure à température ambiante. Le solide formé est essoré, lavé à l'éther froid, puis à l'eau acide (pH 3—4). On obtient 1,6 g de l'ester t-butylique du N(benzoylthio-2 éthyl)-L-Phe. F=170—172°C.

Exemple 13

N-(benzoylthio-2 éthyl)-L-Phe

On mélange 850 mg de l'ester t-butylique de la N-(benzoylthio-2 éthyl)-L-Phe avec 3,31 ml de TFA au bain de glace et on agite pendant 12 heures à température ordinaire. On filtre le précipité blanc et on le lave à l'éther jusqu'à pH 5.

On obtient 620 mg de N-(benzoylthio-2 éthyl)-L-Phe. F=260°C.

Exemple 14

Ester méthylique du N-(benzoylthio-2 éthyl)-L-Phe-L-Leu

On ajoute 0,40 ml de triéthylamine à une solution de 620 mg de N-(benzoylthio-2 éthyl)-L-Phe, tel que préparé à l'exemple 13, et de 254 mg de chlorhydrate de L-Leu $OCH_3$ dans un mélange de 40 ml de $CHCl_3$ et 10 ml de THF. Après 5 minutes, on ajoute 214 mg de HOBT et 288 mg de DCC.

Après agitation pendant 48 heures, on filtre le DCU et après traitement on obtient 250 mg de l'ester méthylique de la N-(benzoylthio-2 éthyl)-L-Phe-L-Leu. Rf=0,42 dans $CHCl_3/Et_2O$, 8/2.

Exemple 15

N-(mercapto-2 éthyl)-L-Phe-L-Leu

Méthode A

250 mg de l'ester méthylique du N-(benzoylthio-2 éthyl)-L-Phe-L-Leu sont dissous dans 15 ml de MeOH. On ajoute 1,1 ml de NaOH 1N, on laisse agiter une nuit à 25°C sous azote. On évapore le méthanol, on ajoute 10 ml $H_2O$ et on acidifie à pH 2—3.

On obtient 65 mg de cristaux blancs de N-(mercapto-2 éthyl)-L-Phe-L-Leu. Rf=0,9 dans $BuOH/AcOH/H_2O$, 4/1/1

Méthode B

5 g de l'ester méthylique de la L-Phe-L-Leu, sous forme de base sont mis en solution dans 50 ml de benzène anhydre dans une ampoule en verre. On ajoute 5 ml de thiiranne, $C_2H_4S$, fraîchement préparé et on scelle l'ampoule.

On chauffe à l'étuve à 80°C pendant 2 heures. Après refroidissement et ouverture de l'ampoule, on élimine par filtration de la solution un précipité gélatineux, puis on concentre le fitrat sous vide jusqu'à obtention d'une huile jaune qui fournit par chromatographie sur colonne d'alumine 2,2, g de l'ester méthylique du N-(mercapto-2 éthyl)-L-Phe-L-Leu. Ce dernier est saponifié sous atmosphère d'azote dans une solution de MeOH et de NaOH 1N. Après acidification et extraction à l'acétate d'éthyle, on obtient 1,19 g de N-(mercapto-2 éthyl)-L-Phe-L-Leu identique au composé obtenu par la méthode A.

Exemple 16

N-(mercapto-2 éthyl)-L-Phe-L-Ala

Obtenu comme à l'exemple 15 (méthode B) en remplaçant L-Leu par L-Ala dans l'ester méthylique de départ, on obtient en partant de 3 g d'ester méthylique 720 mg de N-(mercapto-2 éthyl)-L-Phe-L-Ala.

F=61°C Rf=0,80 $BuOH/MeOH/H_2O$, 4/1/1

Exemple 17

Ester méthylique de la N[(R, S)-acétylthio-3, benzyl-2 propionyl]-L-leucine

On ajoute successivement à une solution refroidie à 0°C de 1,4 g d'acide acétylthio-3 benzyl-2 propionique obtenu par addition de l'acide thioacétique sur l'acide benzylacrylique obtenu selon C. Mannich et K. Ritsert, 1924, Ber 57, 1116 dans 20 ml de THF anhydre, une solution de 1,09 g de chlorhydrate de l'ester méthylique de la L-leucine et de 0,84 ml de triéthylamine dans 20 ml de chloroforme sec, puis une solution de 920 mg d'HOBT dans 20 ml de THF et enfin une solution de 1,22 g de DCC dans 20 ml de $CHCl_3$. On agite 4 heures à 0°C, puis 5 heures à 20°C.

Après filtration du DCU formé, on évapore à sec, reprend le résidu par 40 ml d'acétate d'éthyle. On lave par deux fois avec 20 ml d'eau, une fois avec 20 ml d'une solution saturée de NaCl, deux fois 20 ml d'une solution à 10% d'acide citrique, trois fois avec 20 ml d'une solution saturée de $NaHCO_3$, deux fois avec 20 ml d'eau, une fois avec 20 ml d'une solution saturée de NaCl et on sèche sur $Na_2SO_4$.

On évapore à sec. On obtient 1,9 g d'une huile jaune foncé. Rendement=87%; Rf: 0,86 dans $CHCl_3/MeOH/H_2O$=9/1/1 sat.

HPLC—Vr=28,3 ml dans tampon sulfate $10^{-2}$M(pH=4,3)/$CH_3CN$=55/45.

Exemple 18

N[(R, S)mercapto-3 benzyl-2 propionyl]L-leucine

1,5 g du composé 17 est mis en solution dans 20 ml de méthanol dégazé. On ajoute à 0°C et

sous courant d'azote 8,5 ml d'une solution de soude 1N. On agite 1 heure à 0°C. On évapore le méthanol sous vide et reprend par 20 ml d'eau dégazée et on extrait au chloroforme.

La phase aqueuse est ensuite acidifiée à pH=1 par une solution d'HCl 2N. Il se forme un précipité blanc qui se dissout après addition de 20 ml de méthanol. On ajoute 500 mg de Zn en poudre et on agite 1 heure à 20°C.

On filtre le zinc et on le lave avec deux fois 5 ml de MeOH dégazé. On réunit les phases méthanol-eau et on évapore le méthanol sous vide. La phase aqueuse restante est extraite au chloroforme.

Après évaporation à sec, on obtient 1 g de N(R, S mercapto-3 benzyl-2-propionyl)L-leucine sous forme d'un produit solide blanc. F=50°C; Rdt:79%.

HPLC, Vr=10,56 ml dans tampon acétate $10^{-2}$M (pH 4,3)/$CH_3CN$=60/40.

Analyse élémentaire pour $C_{16}H_{23}NO_3S$:

% calculé: C: 62,1; H: 7,49; N: 4,53; S: 10,36

% trouvé: C: 61,98; H: 7,53; N: 4,46; S: 10,51

RMN ($DMSOd_6$). On observe des signaux séparés pour les deux diastéréosiomères.

HS=2,03 et 2,15 ppm (disparaissent par addition de $D_2O$).
(HS) $CH_2$=2 multiples centrés sur 2,37 et 2,56 ppm.
CH—$CH_2$ ($\phi$)=massif complexe centré sur 2,61 ppm.
$H_\alpha$(Leu): 4,06 et 4,17 ppm
$CH_{2\beta}$Leu=1,30 et 1,44 ppm.
$CH_\gamma$Leu=1,13 et 1,58 ppm.
$CH_3$=0,65 et 0,80 ppm.
NH=8,07 et 8,18 ppm.
H aromatique=7,13 ppm.

Exemple 19

Ester méthylique de la N[(RS) acétyl thio-3 benzyl-2 propionyl] glycine.

A une solution de 2,34 g d'acide acétylthio-3 benzyl-2 propionique dans 20 ml de THF refroidie à 0°C, sont ajoutées successivement une solution de 1,23 g de chlorhydrate de l'ester méthylique de la glycine et de 1,37 ml de triéthylamine dans 25 ml de chloroforme, une solution de 1,50 g d'HOBT dans 15 ml de THF et une solution de 2,22 g de DCC dans 10 ml de $CHCl_3$. On agite 1 heure à 0°C, puis 5 heures à 20°C. Après filtration du DCU formé on traite par le processus décrit pour le composé 17.

On obtient 2,6 g de l'ester méthylique de la N[(R, S) acétylthio-3 benzyl-2 propionyl] glycine sous forme d'une huile jaune pâle.

Rdt=87%; Rf=0,76 dans $CHCl_3$/MeOH/$H_2O$=9/1/sat.

HPLC=Vr=9,12 ml dans tampon acétate $10^{-2}$M (pH: 4,3)/$CH_3CN$=55/45.

Exemple 20

N-[(R, S) mercapto-3 benzyl-2 propionyl] glycine

960 mg du composé de l'exemple 19 sont mis en solution dans 20 ml de MeOH dégazé. On ajoute à 0°C et sous atmosphère d'azote 6,6 ml d'une solution de soude 1N, et on agite 1 heure à 0°C. On évapore sous vide le méthanol, reprend le résidu par 15 ml d'eau. On extrait deux fois par 5 ml de $CHCl_3$.

La phase aqueuse est acidifiée par une solution d'HCl 2N jusqu'à pH: 1. On ajoute 20 ml de MeOH dégazé, puis 300 mg de Zn en poudre et on agite 1 heure à 20°C.

On filtre le zinc et on le lave avec deux fois 5 ml de Me OH. Les phases méthanol-eau sont réunies et le méthanol est évaporé sous vide. La phase aqueuse est alors extraite par trois fois 10 ml de $CHCl_3$. On évapore à sec et on obtient 500 mg de (R, S-mercapto-3 benzyl-2 propionyl) glycine sous forme d'un solide blanc.

F: 138°C; Rdt: 64%.

HPLC, Vr=6,48 ml dans tampon acétate $10^{-2}$M (pH=4,3)/$CH_3CN$=70/30.

Analyse pour $C_{12}H_{15}NO_3S$

% calculé: C: 56,9; H=5,97; N: 5,53; S: 12,66

% trouvé: C: 56,6; H: 6,01; N=5,62; S=12,48

RMN ($DMSOd_6$) une seule série de signaux pour les deux énantiomères.
HS=2,20 ppm (disparaît par addition de $D_2O$).
(HS)$CH_2$=2,34 et 2,57 ppm.
CH—$CH_2$ multiplet centré sur 2,64 ppm.
$CH_2$ (Gly)=3,66 ppm.
H aromatiques=7,17 ppm.
NH=8,28 ppm.

Exemple 21

Ester méthylique de la N[(R, S) acétyl thio-3 benzyl-2 propionyl]-L analine

A une solution refroidie à 0°C de 1,600 g d'acide acétylthio-3 benzyl-2 propionique dans 20 ml de THF anhydre on ajoute successivement une solution de 940 mg de chlorhydrate de l'ester méthylique de la L-alanine et de 1 ml de triéthylamine dans 20 ml de $CHCl_3$, une solution de 1,03 g de HOBT dans 10 ml de THF et 1,4 g de DCC dans 10 ml de $CHCl_3$. On agite 1 heure à 0°C, puis 5 heures à 20°C. On traite comme dans les exemples précédents et on obtient 1,75 g d'ester méthylique de la N-[(R, S) acétyl thio-3 benzyl-2 propionyl]-L alanine sous forme d'une huile jaune. Rdt: 80%; Rf=0,77 dans $CHCl_3$/MeOH/eau: 9/1/sat.

HPLC, Vr=10,32 dans tampon acétate $10^{-2}$M (pH=4,3)/$CH_3CN$=55/45.

Exemple 22

N-[(RS) mercapto-3 benzyl-2 propionyl]L alanine

1,54 g du composé de l'exemple 21 sont mis en solution dans 20 ml de méthanol dégazé, on ajoute à 0°C et sous azote 10 ml d'une solution de soude 1N. On agite 2 heures à 0°C. On évapore le méthanol sous vide et on reprend par 20 ml d'eau. On extrait deux fois la phase aqueuse alcaline par 5 ml de chloroforme. La phase aqueuse est acidifiée par HCl 2N jusqu'à pH=1. On ajoute alors 20 ml de MeOH dégazé et 300 mg de poudre de Zn. On agite 1 heure à 20°C. On filtre le poudre de zinc et on la lave deux fois avec du méthanol. Les phases aqueuses et méthanolique sont réunies. Le méthanol est évaporé sous vide et la phase aqueuse restante est extraite au chlöroforme. On évapore à sec. On obtient 1,07 g de N-[(R, S) mercapto-3 benzyl-2 propionyl]L alanine sous forme d'un solide blanc.

F<50°C; Rdt: 84%.

HPLC, Vr=5,4 ml dans tampon acétate $10^{-2}$M (pH=4,3)/$CH_3CN$=65/35.

Analyse pour $C_{13}H_{17}NO_3S$:

| | |
|---|---|
| % calculé: | C: 58,40; H: 6,41; N: 5,24; S: 11,99 |
| % trouvé: | C: 58,23; H: 6,60; N: 5,31; S: 11,64 |

RMN ($DMSOd_6$) on observe 1 jeu de signaux pour chaque diastéréoisomère.

HS=2,06 et 2,12 ppm (disparaissent par addition de $D_2O$).

(HS)$CH_2$ multiplets centrés sur 2,34 et 2,58 ppm.

(HS)$CH_2$ ($\phi$) massif complexe centré sur 2,75 ppm.

NH=8,17 et 8,27 ppm.

$H_2$Ala: 4,08 et 4,15 ppm.

$CH_3$Ala: 1,05 et 1,20 ppm.

H aromatiques: 7.17 ppm.

Exemple 23

Ester méthylique de la O-benzyl N-[(R, S) benzoylthio-3 benzyl-2 propionyl]L-sérine

A une solution refroidie à 0°C de 500 mg d'acide benzoylthio-3 benzyl-2 propionique dans 10 ml de THF anhydre, sont ajoutées successivement une solution de 408 mg de chlorhydrate de l'ester méthylique de la O-benzyl-L-sérine et de 0,24 ml de triéthylamine dans 6 ml de $CHCl_3$, une solution de 250 mg de HOBT dans 5 ml de THF anhydre et enfin une solution de 376 mg de DCC dans 5 ml de $CHCl_3$. On agite 1 heure à 0°C et 5 heures à 20°C. Après filtration du DCU formé, on traite comme dans les exemples précédents et on obtient 720 mg d'ester méthylique de la O-benzyl N-[(RS)benzoylthio-3 benzyl-2 propionyl]L-sérine sous forme d'un solide blanc.

Rdt: 88%; F=62—64°C; Rf=0,84 dans $CHCl_3$/MeOH/eau=9/1/sat.

HPLC, Vr=9,84 ml dans tampon acétate $10^{-2}$M (pH: 4,3)/$CH_3CN$=30/70.

Exemple 24

O-benzyl, N-[(R, S) mercapto-3 benzyl-2 propionyl]L-sérine

660 mg du composé de l'exemple 23 sont mis en solution dans 10 ml de MeOH dégazé. On ajoute à 0°C et sous azote, 3 ml d'une solution de soude 1N. On agite 1 heure à 0°C. On évapore le méthanol sous vide et reprend par 10 ml d'eau. On extrait deux fois au chloroforme. La phase aqueuse est acidifiée par HCl 2 N jusqu'à pH: 1. On ajoute 10 ml de méthanol et 150 mg de poudre de Zn. On agite 1 heure à 20°C. On filtre le zinc, on le lave, avec deux fois 5 ml de MeOH. Les phases aqueuse et méthanolique sont réunies et le méthanol est évaporé sous vide. La phase aqueuse est extraite au chloroforme. Après évaporation, on obtient 350 mg de la O-benzyl, N-[(R, S) mercapto-3 benzyl-2-propionyl] L-sérine sous forme d'une huile jaune très pâle. Rdt: 70%.

HPLC, Vr: 6,96 ml dans tampon acétate $10^{-2}$M (pH: 4,3)/$CH_3CN$=55/45.

RMN (DMSO-$d_6$):

HS=2,02 et 2,12 ppm (disparaissent par addition $D_2O$) (HS)$CH_2$=2,33 et 2,56 ppm.

$CHCH_2$ ($\phi$)=massifs centrés sur 2,70 ppm.

HαSer=4,37 et 4,44 ppm.
$CH_2$βSer=3,46 et 3,62 ppm.
$OCH_2$=4,32 et 4,42 ppm.
H aromatiques=7,17 ppm.
NH=8,25 et 8,33 ppm.

Analyse pour $C_{20}H_{23}NO_4S$

| | |
|---|---|
| % calculé: | C: 64,32; H: 6,21; N: 3,75; S: 8,58 |
| % trouvé: | C: 64,30; H: 6,31; N: 3,87; S: 8,66 |

Exemple 25

Ester méthylique de la N[(R, S) acétyl thio-3 p.fluoro benzyl-2 propionyl]L-leucine

On ajoute successivement à une solution refroidie à 0°C de 1,54 g d'acide acétyl thio-3 p.fluorobenzyl-2 propionique dans 20 ml de THF anhydre, une solution de 1,15 g de chlorhydrate de l'ester méthylique de la L-leucine et de 0,85 ml de triéthylamine dans 20 ml de chloroforme sec, puis une solution de 930 mg d'HOBT dans 20 ml de THF et enfin une solution de 1,24 g de DCC dans 20 ml de $CHCl_3$. On agite 1 heure à 0°C, puis 5 heures à 20°C. Après filtration du DCU formé et traitement comme dans l'exemple 17, on obtient 1,95 g d'huile jaune foncée. Rdt: 88%, Rf: 0,87 $CHCl_3$/MeOH/$H_2O$=0/1/sat.

HPLC, Vr=30 ml dans tampon acétate $10^{-2}$M (pH=4,3)/$CH_3CN$=55/45.

Exemple 26

N-[(R, S) mercapto-2 p.fluorobenzyl-3 propionyl] L-leucine

1,8 g du composé 25 est mis en solution dans 20 ml de méthanol dégazé. On ajoute à 0°C et sous courant d'azote 9,8 ml d'une solution de soude 1N. On agite 1 heure à 0°C. On évapore le méthanol sous vide et on reprend par 20 ml d'eau dégazée et on extrait au chloroforme.

La phase aqueuse est ensuite acidifiée à pH=1 par une solution d'HCl 2N. Il se forme un précipité blanc qui se dissout après addition de 20 ml de méthanol. On ajoute 500 mg de Zn en poudre et on agite 1 heure à 20°C. On filtre le zinc et on le lave avec deux fois 5 ml de MeOH dégazé. Les phases méthanoliques et aqueuses sont réunies et le méthanol est évaporé sous vide.

La phase aqueuse est alors extraite par trois fois 10 ml de $CHCl_3$. On évapore à sec et on obtient 1,08 g du composé indiqué dans le titre sous forme d'un solide blanc.

Rdt: 68%; F<50°C.

HPLC, Vr: 11 ml dans tampon acétate $10^{-2}$ (pH=4,3)/$CH_3CN$—60/40.

RMN (DMSO-$d_6$).

HS=2,04 et 2,12 ppm (disparaissent par addition de $D_2O$) (HS)$CH_2$=2 multiplets centrés sur 2,40 et 2,58 ppm.

$CHCH_2$ (ϕ) massif complexe centré sur 2,63 ppm.
HαLeu=4,06 et 4,17 ppm.
$CH_2$βLeu=1,31 et 1,44 ppm.
CHγLeu=1,13 et 1,58 ppm.
$CH_3$=0,65 et 0,81 ppm.
NH=8,08 et 8,19 ppm.
H aromatiques=2 massifs centrés sur 7,28 et 7,95 ppm.

Analyse pour $C_{16}H_{22}O_3NFS$

| | |
|---|---|
| % calculé: | C: 58,70; H: 6,78; N=4,28; S: 9,78 |
| % trouvé: | C: 58,55; H: 6,60; N: 4,32; S: 9,98 |

Exemple 27

N[(S) benzoylthio-2 phényl-3 propionyl]-L-leucine

On ajoute à une solution de 11,2 g d'acide R, bromo-2 phényl-3 propionique dans 30 ml de THF, 8,9 g de chlorhydrate de l'ester méthylique de la L-leucine dissous dans un mélange de 100 ml de $CHCl_3$ et 6,86 ml de $NET_3$. A la solution obtenue on ajoute 7,5 g d'HOBT et 10,1 g de DCC. Après agitation 1 heure à 0°C, puis 12 heures à 25°C, on filtre le DCU formé et traite par le processus habituel. On obtient par évaporation de l'acétate d'éthyle, 12,5 g de cristaux blancs d'ester méthylique de la N[(R) bromo-2 phényl-3 propionyl]-L-leucine. F=121°C.

On ajoute à 0°C, 30 ml de NaOH 1N à une solution de 10,5 g du composé précédent dans 100 ml de MeOH. Après agitation 1 heure à 0°C, puis 4 heures à 25°C, on acidifie par HCl. Le précipité est recueilli, puis cristallisé dans l'acétate d'éthyle. On obtient 9,07 g de N[(R)bromo-2 phényl-3 propionyl]-L-leucine. F=158°C.

1,76 g d'acide thiobenzoïque dissous dans 40 ml d'eau, en présence de 2,17 g de $K_2CO_3$ sont ajoutés à un mélange de 7,1 g de N[(R)bromo-2 phényl-3 propionyl]-L-leucine dans 30 ml d'eau et 1,74 g de bicarbonate de Na. On agite 12 heures à 25°C. On acidifie par HCl 6N, ce qui fournit une huile jaune pâle qui est extraite par de l'acétate d'éthyle. Après lavage à l'eau, on sèche la phase organique, évapore à sec. On obtient 7,9 g de N[(S)benzoyl-thio-2 phényl-3 propionyl]-L-leucine sous forme d'huile qui cristallise lentement.

F=74°C—78°C; Rf=0,80 dans BuOH/MeOH/$H_2O$, 4/1/1.

Exemple 27

N[(S)mercapto-2 phényl-3 propionyl]-L-leucine

2 g de N(S,benzoylthio-2 phényl-3 propionyl)-L-leucine en solution dans 10 ml de MeOH sont agités à 0°C pendant 1 heure avec 10 ml de NaOH 1N, puis 4 heures à 25°C sous azote. On évapore le méthanol, reprend par l'eau et acidifie à pH=1 par HCl 1N. Il se forme un précipité gommeux que l'on extrait à l'acétate d'éthyle. La phase organique est séchée, évaporée sous vide. On obtient 1,45 g de N[(S)mercapto-2 phényl-3 propionyl]-L-leucine qui cristallise lentement. F=67—71°C.

Exemple 29

Ester méthylique de la N[(S)acétylthio-2 phényl-3 propionyl]-L-leucine

3 g de l'acide S, acétylthio-2 phényl-3 propionique (obtenu par action de l'acide thioacétique sur l'acide R, bromo-2 phényl-3 propionique) en solution dans 30 ml de $CHCl_3$ sont ajoutés à une solution de 2,5 g de chlorhydrate de l'ester méthylique de la L-leucine. On ajoute 5 ml de $NEt_3$, puis 2 g de HOBT et 2,8 g de DCC. Après agitation 1 heure à 0°C, puis 12 heures à 25°C, on filtre le DCU formé et traite par le processus habituel. On obtient 4,1 g de l'ester méthylique de la N-[(S)acéthylthio-2 phényl-3 propionyl]-L-leucine sous forme d'huile.

Rf: 0,85 dans $CHCl_3$/MeOH/$H_2O$, 9/1/Sat.

Exemple 30

Ester méthylique de la N[(S)mercapto-2 phényl-3 propionyl]-L-leucine.

2 g du composé de l'exemple 29 sont agités 12 heures dans une solution de méthanol saturée d'HCl, sous azote. On évapore à sec, reprend par l'eau et extrait à l'éther. On sèche la phase organique, évapore à sec. On obtient 750 mg de l'ester méthylique de la N[(S)mercapto-2 phényl-3 propionyl]-L-leucine sous forme d'huile.

Rf: 0,70 dans $CHCl_3$/$Et_2O$, 5/5.

Exemple 31

N[(S)acétylthio-2 phényl-3 propionyl]-L-leucine

Ce composé est obtenu comme à l'exemple 27 en remplaçant l'acide thiobenzoïque par l'acide thioacétique. On obtient une huile jaune pâle qui cristallise lentement.

F=50°C; Rdt: 84%; Rf=0,80 dans BuOH/MeOH/$H_2O$=4/1/1.

Exemple 32

N[(S)mercapto-2 phényl-3 propionyl]-L-Alanine

Obtenu en suivant les méthodes successivement appliquées dans les exemples 27 et 28 en remplaçant l'ester méthylique de la L-leucine par l'ester méthylique de la L-alanine. On obtient ainsi le N[(S)mercapto-2 phényl-3 propionyl]-L-Ala, cristaux blancs. F=42°C.

Exemple 33

N[(S)acétylthiométhylthio-2 phényl-3 propionyl]-L-leucine

On ajoute 0,42 ml d'acétylthio chlorométhane en solution dans MeOH à un mélange de 1,1 g de N[(S)mercapto-2 phényl-3 propionyl]-L-leucine obtenue selon l'exemple 28 et de 552 mg de $K_2CO_3$ dans l'eau. On agite 20 heures à température ordinaire, évapore à sec et reprend par l'acétate d'éthyle. On sèche, évapore. On obtient 252 mg de N(acétylthiométhylthio-2 phényl-3 propionyl)-L-leucine sous forme d'huile après purification par chromatographie sur colonne d'alumine dans le mélange $CHCl_3$/MeOH, 7/3. Rf=0,77 $CHCl_3$/MeOH, 5/3.

Exemple 34

N[(S)benzoylthio éthylthio-2 phényl-3 propionyl]-L-leucine

1,33 g de N[(S)mercapto-2 phényl-3 propionyl]-L-leucine obtenue selon l'exemple 28 sont dissous dans 30 ml d'eau en présence de 685 mg de $K_2CO_3$. On ajoute à 0°C, 1,32 g de benzoylthio-1 bromo-2 éthane (obtenu par action directe du bromure de benzoyle sur le thiiranne selon B. Hansen. Acta. Chem. Scand *11*, 537 (1957) en solution dans 10 ml de MeOH. On agite 48 heures à 25°C. On filtre d'un léger précipité cotonneux et on acidifie à pH=2 par HCl 6N. On extrait trois fois avec 20 ml d'acétate d'éthyle, sèche et évapore à sec. On obtient 1,50 g du composé attendu à l'état d'huile qui cristallise lentement.

F=62—68°C.

Exemple 35

N[(S)mercapto éthylthio-2 phényl-3 propionyl]-L-leucine

On dissout 800 mg du composé de l'exemple 34 dans 10 ml de MeOH et on ajoute sous azote 3,4 ml de NaOH 1N en 30 minutes. On laisse 4 heures à 25°C, toujours sous azote. On ajoute 20 ml d'$H_2O$, évapore MeOH, filtre et acidifie par HCl 1N jusqu'à pH=1. On extrait avec trois fois 20 ml d'éther, sèche et évapore à sec. On obtient 544 mg d'une huile incolore. Rf=0,92 dans BuOH/AcOH/$H_2O$, 4/1/1, correspondant à N[(S)mercapto éthylthio-2 phényl-3 propionyl]-L-leucine.

Exemple 36

N[(S)mercapto éthylthio-2 phényl-3 propionyl]-L-alanine

En remplaçant dans l'exemple 34 la N[(S)mercapto-2 phényl-3 propionyl]-L-leucine par la N[(S)mercapto-2 phényl-3 propionyl]-L-alanine obtenue selon l'exemple 32, on obtient la N[(S)benzoylthio éthylthio-2 phényl-3 propionyl]-L-alanine. Ce dérivé, traité comme dans l'exemple 35, fournit la N[(S)mercapto éthylthio-2 phényl-3 propionyl]-L-alanine. Huile épaisse qui cristallise lentement.

F=34°C.

Exemple 37

Préparation de N- Boc, N'-(éthoxycarbonyl-2 propionyl) diamino-1;1 phényl-2 éthane

a) N-t butoxycarbonyl, N-benzyloxycarbonyl diamino-1,1-phényl-2 éthane

1,5 g de Boc L-Phe-azide sont portés à 80°C pendant 1 heure dans 10 ml de toluène anhydre. Il se forme un isocyanate qui n'est pas isolé. Après refroidissement de la solution, on ajoute 0,55 ml d'alcool benzylique, puis on chauffe à nouveau à 80°C pendant 1 heure. Après refroidissement, la solution laisse cristalliser 0,9 g de N-Boc, N'-Z diamino-1,1 phényl-2 éthane. F=165°C.

b) N-Boc diamino-1,1 phényl-2 éthane

0,4 g de N-Boc, N'-Z diamino-1,1 phényl-2 éthane sont mis en solution dans 10 ml de méthanol en présence de 25 mg de Pd/C. L'hydrogénation à pression ordinaire fournit après filtration et évaporation à sec 0,3 g de N-Boc diamino-1,1 phényl-2 éthane. Solide jaune pâle. F=40—50°C.

c) Produit énoncé dans le titre

270 mg de monoester méthylique de l'acide méthyle malonique (R, S) en solution dans 10 ml de THF sont ajoutés à une solution de 436 mg de N-Boc diamino-1,1 phényl-2 éthane, 283 mg HOBT et 381 mg DCC dans 10 ml $CHCl_3$. Après 4 heures à température ambiante, on filtre le DCU formé et traite la solution dans les conditions habituelles du couplage peptidique. On obtient ainsi 544 mg de solide jaune pâle. Rf=0,80 dans $CHCl_3$/MeOH/$H_2O$,9/1/Sat, de N-Boc, N'-(éthoxycarbonyl-2 propionyl)] diamino-1,1 phényl-2 éthane (SR+SS).

Exemple 38

N-Boc, N'(carboxy-2 propionyl) diamino-1,1 phényl-2 éthane

200 mg du composé obtenu à l'exemple 37 sont mis en solution dans 10 ml de MeOH. On ajoute 1,5 ml NaOH 1N, agite 4 heures à température ambiante, évapore MeOH, reprend par $H_2O$ et acidifie à pH=2. On extrait par l'acétate d'éthyle, sèche et évapore à sec. On obtient 130 mg de cristaux blancs de N-Boc, N'(carboxy-2 propionyl) diamino-1,1 phényl-2 éthane (SR+SS). F=170—171°C.

Exemple 39

N-(carboxy-2 propionyl) diamino-1,1 phényl-2 éthane

100 mg du composé obtenu à l'exemple 38 sont agités avec 0,4 ml de TFA pendant 1 heure à 25°C. Après évaporation à sec et reprise par l'éther éthylique, on obtient 60 mg de N-(carboxy-2 propionyl) diamino-1,1 phényl-2 éthane (RR+RS). Rf=0,35 dans BuOH/AcOH/$H_2O$, 4/1/1.

Exemple 40

N-carboxyméthyl, N'(carboxy-2 propionyl) diamino-1,1 phényl-2 éthane

300 mg du composé obtenu dans l'exemple 37 sont agités 1 heure à 0°C avec 1 ml de TFA. Après évaporation à sec et addition d'éther, le solide blanc obtenu est séché sous vide, puis mis en suspension dans du benzène anhydre et chauffé à reflux 12 heures avec 0,1 ml de bromoacétate d'éthyle.

Après filtration, on recueille une huile jaune pâle de N-éthoxycarbonylméthyl, N'(éthoxycarbonyl-2 propionyl) diamino-1,1 phényl-2 éthane, qui est saponifié sans purification pour conduire à 52 mg de N-carboxyméthyl, N'(carboxy-2 propionyl) diamino-1,1 phényl-2 éthane (RR+RS).

Rf=0,42 dans BuOH/AcOH/$H_2O$, 4/1/1.

Exemple 41

N-(mercapto-2 éthyl), N'(carboxy-2 propionyl) diamino-1,1 phényl-2 éthane (RR+RS)

Obtenu par action directe du thiiranne en tube scellé sur 50 mg du composé de l'exemple 39 en suivant la méthode décrite dans l'exemple 15 (méthode B). F=180—190°C.

Exemple 42

Boc amino-2 phényl-3 carboxyméthylthio-1 propane

On ajoute 2,3 g de l'ester p.toluène sulfonique du Boc-L phénylalaninol (obtenu par réduction de la t-Boc-L Phe en alcool, puis action du chlorure de tosyle) dissous dans 50 ml de MeOH, à une solution aqueuse du sel disodique de l'acide thioglycolique. Après reflux pendant 12 heures, on filtre, acidifie la solution et extrait à l'acétate d'éthyle. On obtient 700 mg d'une huile épaisse qui est chromatographiée sur colonne d'alumine avec un mélange $Et_2O$/MeOH, 7/3.

On recueille 550 mg de Boc amino-2 phényl-3 carboxy méthylthio-1 propane.

F=95°C. Rf=0,63 dans $CHCl_3$/MeOH 7/3.

Exemple 43

Trifluoroacétate de (amino-2 phényl-3 carboxyméthylthio-1) propane

400 mg du composé décrit à l'exemple 42 sont agités 1 heure à 25°C avec 1 ml de TFA. Après évaporation à sec et reprise par l'éther, on obtient 300 mg du trifluoroacétate de (amino-2 phényl-3 carboxyméthylthio-1) propane sous forme de poudre blanche. Rf=0,41 dans BuOH/MeOH/$H_2O$ 4/1/1.

Exemple 44

Carboxyméthylamino-2 phényl-3 carboxyméthylthio-1 propane

250 mg du composé décrit à l'exemple 43 sont mis en suspension dans 10 ml de benzène anhydre. On ajoute 0,15 ml de triéthylamine et 0,21 ml de bromoacétate d'éthyle. La solution obtenue est mise au reflux pendant 10 heures. On filtre, sèche et évapore à sec. L'huile obtenue est chromatographiée sur colonne de gel de silice dans le mélange $Et_2O$/MeOH, 8/2.

On obtient 125 mg de carboxyméthyl amino-2 phényl-3 carboxyméthylthio-1 propane. Rf=0,57 dans $CHCl_3$/MeOH, 8/2.

Exemple 45

(mercapto-2 éthylamino)-2 phényl-3 carboxyméthylthio-1 propane

150 mg du composé décrit à l'exemple 43 sont traités comme à l'exemple 15 (méthode B) pour conduire à 42 mg de (mercapto-2 éthyl amino)-2 phényl-3 carboxyméthyl thio-1 propane. F=201°C.

Exemple 46

Ester méthylique de la N-(Boc amino-2 phényl-3 propyl-1) L-leucine

On ajoute 2 g de l'ester p.toluène sulfonique du Boc-L Phe-alaninol à une solution de 1,4 g de l'ester méthylique de la L-leucine (sous forme base) dans 10 ml de dioxanne. On porte au reflux pendant 10 heures. On évapore la solution à sec et reprend par de l'éther. Un précipité se forme qui est filtré, puis séché. On obtient ainsi 1,1 g de l'ester méthylique N-(Boc amino-2 phényl-3 propyl-1) L-leucine. F=61°C.

Exemple 47

N(carboxyméthylamino-2 phényl-3 propyl-1) L-leucine

500 mg du composé décrit à l'exemple 46, sont agités 1 heure avec 2 ml de TFA. Après évaporation à sec, et addition d'éther, on recueille un solide blanc qui est séché sous vide. On place ce produit sans purification en suspension dans 20 ml de benzène anhydre et on ajoute 0,2 ml de triéthylamine et 0,15 ml de bromoacétate d'éthyle. Après reflux pendant 15 heures, la solution est filtrée, puis évaporée à sec. L'huile obtenue est purifiée par chromatographie sur colonne d'alumine avec un mélange $Et_2O$/MeOH, 9/1. On obtient 120 mg de l'ester méthylique de la N(carboxyméthylamino-2 phényl-3 propyl-1) L-leucine. F=40—50°C.

On dissout 100 mg de ce produit dans 20 ml de méthanol et ajoute NaOH jusqu'à pH=9. Après agitation pendant 2 heures, on acidifie par HCl (pH=4), puis extrait à l'acétate d'éthyle et évapore à sec.

On obtient 23 mg de N-(carboxyméthylamino-2 phényl-3 propyl-1)-L-leucine. F=147—151°C.

Exemple 48

N[(mercapto-2 éthylamino)-2 phényl-3 propyl-1] L-leucine

Obtenu comme à l'exemple 15 (méthode B), en remplaçant l'ester méthylique de la L-Phe-L-Leu par 200 mg de l'ester méthylique de la N(amino-2 phényl-3 propyl-1) L-leucine obtenue à partir du composé de l'exemple 46 par action du TFA.

Après action du thiiranne et saponification sous azote, on obtient 32 mg de N[(mercapto-2 éthylamino)-2 phényl-3 propyl-1] L-leucine. F=48°C.

Exemple 49

Ester méthylique de [éthoxycarbonylméthyl]-1 L-prolyl-L-alanine

On ajoute à une suspension de 5 g (16 mmoles) de l'ester méthylique et trifluoroacétate de L-prolyl-L-alanine dans du benzène anhydre, 1,76 ml de triéthylamine dans 2 g d'acétate d'éthyle. La solution est portée au reflux pendant 18 heures. Le produit réactionnel est traité par le processus décrit à l'exemple 1 pour obtenir 2,05 g (rendement 45%) d'un produit huileux. Rf ($CHCl_3$/MeOH/Eau=9/1/Sat)=0,73.

Exemple 50

[Carboxyméthyl]-1 L-propyl-L-alanine

Ce composé est obtenu par le même processus que celui décrit à l'exemple 2. A partir de 2 g de l'ester méthylique de [éthoxyméthyl]-1 L-prolyl-L-alanine, on obtient 900 mg de [carboxyméthyl]-1 L-prolyl-L-alanine (rendement 50%). Point de fusion: 158°C. Rf (BuOH/MeOH/$H_2O$=4/1/1)=0,42.

Exemple 51

Préparation de l'ester benzylique de N[phénylacétylthio-3 benzyl-2 propionyl] glycine

a) Acide mercapto-3 benzyl-2 propionique

A une solution d'acide acétylthio-3 benzyl-2 propionique (2,7 g, 11 mmoles) dans 20 ml de MeOH dégazé on ajoute, à 0°C, une solution dégazée de NaOH (1 g, 25 mmoles) dans 20 ml d'eau dégazée. Après 1 heure à 0°C, on agite le mélange à température ambiante pendant 4 heures. Les solvants sont évaporés, le résidue est dissous dans de l'eau dégazée (20 ml) et extrait par $CHCl_3$ (10 ml). On ajoute à la couche aqueuse, à 0°C, 20 ml de MeOH dégazé, 13 ml de HCl3N et 1 g de Zn en poudre. Le mélange est agité pendant 1 h. 30 à la même température. Après filtration, on évapore MeOH et la couche aqueuse est extraite avec du chloroforme dégazé (3×20 ml). La couche chloroformique est séchée sur sulfate de sodium et évaporée. Une huile incolore est obtenue (1,9 g, 85%). Rf ($CHCl_3$/MeOH: 7/3)=0,67.

b) Acide phénylacétylthio-3 benzyl-2 propionique

On ajoute à une solution refroidie à la glace d'acide mercapto-3 benzyl-2 propionique (600 mg, 3 mmoles) dans 5 ml d'eau dégazée, 6,4 ml de NaOH 1N et 0,45 ml (3,4 mmoles) de $\phi CH_2COCl$. Après 15 minutes à 0°C, le mélange est agité pendant 3 heures à température ambiante. La solution est acidifiée à pH 4 par HCl 1N et extraite par de l'éther éthylique (3×5 ml).

La couche éthérée est lavée et séchée sur sulfate de sodium. Après évaporation, 960 mg de solide blanc sont obtenus (rendement 99%). Rf ($CHCl_3$/MeOH: 7/3)=0,77.

c) Ester benzylique de N[phénylacétylthio-3 benzyl-2 propionyl] glycine

Il est obtenu par un procédé analogue à celui décrit à l'exemple 17.

A partir de 560 mg (1,78 mmole) d'acide phénylacétylthio-3 benzyl-2 propionique et 600 mg (1,78 mmole) d'ester benzylique et p.tosylate de glycine, on obtient 660 mg (rendement 80%) du composé annoncé dans le titre sous forme d'un solide blanc. Point de fusion: 82±1°C. Rf ($CHCl_3$/MeOH/eau: 9/1/Sat)=0,77. Chromatographie à haute pression sous phase liquide (HPLC) $V_r$=7,4 ml dans $CH_3CN$/tampon $AcONH_4$ ($10^{-2}$M, pH 4,2)=70/30.

Exemple 52

Ester benzylique de N[benzoylthio-3 benzyl-2 propionyl] glycine

Ce composé est obtenu d'une manière analogue à celle décrite dans l'exemple 17.

On obtient à partir de 475 mg (1,58 mmole) d'acide benzoylthio-3 benzyl-2 propionique et 532 mg (1,58 mmole) d'ester benzylique et p.tosylate de glycine, 680 mg (rendement 90%) du composé indiqué dans le titre sous forme d'un solide blanc recristallisé dans l'éther éthylique. Point de fusion=80±1°C. Rf ($CHCl_3$/MeOH/eau: 9/1/Stat)=0,85.

HPLC $V_r$=7,7 ml dans $CH_3CN$/tampon $AcONH_4$ ($10^{-2}$M, pH 4,2)=70/30.

Exemple 53

Ester benzylique de N[acétylthio-3 benzyl-2 propionyl] glycine

A une solution agitée et refroidie à la glace de 2,85 g (12 mmoles) d'acide acétylthio-3 benzyl-2 propionique dans du tétrahydrofuranne (20 ml), on ajoute successivement un mélange de 4,04 g (12 mmoles) de p.tosylate de Gly-OBzl et 1,71 ml de triéthylamine dans $CHCl_3$ (20 ml), une solution de 1,83 g (12 mmoles) de HOBT dans du tétrahydrofuranne (15 ml) et une solution de 2,63 g (12,8 mmoles) de DCC dans $CHCl_3$ (10 ml).

Après 1 heure on laisse le mélange revenir à température ambiante et on l'agite pendant 16 heures. On opère suivant le même mode opératoire que celui décrit à l'exemple 17 pour obtenir 3 g d'un solide blanc recristallisé dans l'éther éthylique (rendement 75%). Point de fusion=89±1°C. Rf ($CHCl_3$/MeOH/eau: 9/1/Sat)=0,80.

Exemple 54

Ester p.fluoro benzylique de N[acétylthio-3 benzyl-2 propionyl] glycine

Ce composé est obtenu par un processus analogue à celui décrit à l'exemple 53. A partir de 1 g (4,2 mmoles) d'acide acétylthio-3 benzyl-2 propionique et de 1,49 g (4,2 mmoles) d'ester p.fluoro benzylique et p.tosylate de glycine, on obtient 1,18 g (rendement 70%) d'un solide blanc recristallisé dans l'éther éthylique. Point de fusion 72±1°C. Rf ($CHCl_3$/MeOH/eau: 9/1/Sat)=0,75.

Exemple 55

Ester-trifluoro-2,2,2 éthylique de N[acétylthio-3 benzyl-2 propionyl] glycine

Le composé indiqué dans le titre est obtenu par un processus analogue à celui décrit à l'exemple 53.

A partir de 1 g (4,2 mmoles) d'acide acétylthio-3 benzyl-2 propionique et 1,14 g (4,2 mmoles) d'ester trifluoro-2,2,2 éthylique et trifluoroacétate de glycine, on obtient 1,2 g (rendement 82%) d'un produit huileux qui cristallise lentement. Point de fusion 56±1 °C.

Rf ($CHCl_3$/MeOH/eau: 9/1/Sat)=0,77.

Exemple 56

Benzylamide de N[acétylthio-3 benzyl-2 propionyl]glycine

Le composé indiqué dans le titre est obtenu suivant le processus décrit à l'exemple 53.

A partir de 1 g (4,2 mmoles) de l'acide acétylthio-3 benzyl-2 propionique et 1,17 g (4,2 mmoles) de benzylamide et trifluoroacétate de glycine, on obtient 1,32 g (rendement 79%) d'un solide blanc. Point de fusion=62 °C. Rf ($CHCl_3$/MeOH/eau: 9/1/Sat)=0,42.

Exemple 57

Préparation de l'ester benzylique de N[mercapto-3 benzyl-2 propionyl] glycine

a) Acide dithio-1,1' bis (benzyl-2 propionique-3)

A une solution d'acide acétylthio-3 benzyl-2 propionique (13 g) dans 70 ml de MeOH on ajoute, à 0°C, une solution de 4,9 g de NaOH dans 50 ml d'eau. Après 1 heure à 0°C, le mélange est agité pendant 4 heures à température ambiante. Une solution d'iode dans l'éthanol (0,3 M) est ajoutée goutte à goutte jusqu'à ce qu'une coloration jaune persistante soit obtenue. Les solvants sont évaporés et le résidu est dissous dans l'eau. La couche aqueuse est acidifiée à pH 2 par HCl 3N et extraite par l'éther diéthylique.

La couche éthérée est lavée, séchée et évaporée. Un produit huileux (10 g, rendement 95%) qui cristallise lentement est obtenu. Rf (BuOH/AcOH/Fau: 4/1/1)=0,81.

b) Ester benzylique de N[mercapto-3 benzyl-2 propionyl] glycine

A une solution de 1 g (2,56 mmoles) d'acide dithio-1,1' bis (benzyl-2 propionique-3) dans du tétrahydrofuranne on ajoute successivement un mélange de 1,73 g (5,12 mmoles) d'ester benzylique et p.tosylate de glycine et 0,72 ml de triéthylamine, une solution de 0,77 g (5,12 mmoles) de HOBT dans du tétrahydrofuranne et une solution de 1,1 g (5,12 mmoles) de DCC dans $CHCl_3$. Le produit réactionnel est traité comme à l'exemple 17. 1,53 g (rendement 87%) d'ester benzylique de 1,1'-dithio bis (benzyl-2 propionyl-3) bis glycine sont obtenus. Rf. ($CHCl_3$/MeOH/$H_2O$: 9/1/Sat)=0,91.

Le composé indiqué dans le titre (b) est obtenu en agitant 2 heures l'ester benzylique précédemment obtenu dans un mélange de MeOH et de HCl3N avec 500 mg de zinc en poudre. Après filtration et extraction par $CHCl_3$, un produit huileux est obtenu. (Rendement 76%). Rf ($CHCl_3$/MeOH/$H_2O$: 9/1/Sat)=0,76.

HPLC $V_r$=5 ml dans $CH_3CN$/tampon $ACONH_4$ ($10^{-2}$M, pH 4,2) 70/30.

Exemple 58

Ester p.fluorobenzylique de N[mercapto-3 benzyl-2 propionyl] glycine

Le composé indiqué dans le titre est obtenu par un processus analogue à celui décrit dans l'exemple 57 à partir d'ester p.fluorobenzylique et p.tosylate de glycine au lieu de l'ester benzylique et p.tosylate de glycine.

On obtient un produit huileux (rendement 75%).

Rf ($CHCl_3$/MeOH/$H_2O$:9/1/Sat)=0,74

Exemple 59

Estertrifluoro-2,2,2 éthylique de N[mercapto-3 benzyl-2 propionyl] glycine

Le composé indiqué dans le titre est obtenu par un processus analogue à celui décrit à l'exemple 57, à partir d'ester trifluoro-2,2,2 éthylique et trifluoroacétate de glycine à la place de l'ester benzylique et p.tosylate de glycine.

On obtient un produit huileux (rendement 78%).

Rf ($CHCl_3$/MeOH/$H_2O$: 9/1/Sat)=0,80.

Exemple 60

Benzylamide de N[mercapto-3 benzyl-2 propionyl] glycine

Le composé indiqué dans le titre est obtenu par un processus analogue à celui décrit à l'exemple 57, à partir de benzylamide et trifluoroacétate de glycine au lieu de l'ester benzylique et p.tosylate de glycine. On obtient un produit huileux qui cristallise lentement (rendement 90%). Rf ($CHCl_3$) /MeOH/eau: 9/1/Sat)=0,34.

Les résultats des études biologiques et pharmacologiques rapportées ci-après mettent en évidence les intéressantes propriétés inhibitrices de l'enképhalinase, antalgique et hypotensive.

La présente invention a donc encore pour objet un médicament ayant notamment des propriétés inhibitrices de l'enképhalinase, antalgique et hypotensive, caractérisé en ce qu'il contient à titre de principe actif un composé de formule I ou un sel d'addition avec un acide ou une base pharmaceutiquement acceptable de ce composé.

A) Etude biologique

Dosage de l'activité "enképhalinasique" (enképhaline dipeptidylcarboxypeptidasique) et détermination de l'effet des inhibiteurs.

La préparation enzymatique utilisée est une fraction membranaire provenant du striatum de rat ou de souris.

Cette fraction est obtenue par homogénéisation à 4°C dans 20 volumes de tampon Tris-HCl 0,05 M (pH 7,4) suivie de deux centrifugations successives (1000 g×min. et 200.000 g×min.) au terme desquelles le culot de la deuxième centrifugation est retenu. Il est lavé par remise en suspension dans 10 ml du tampon suivie de centrifugation (200.000 g×min.) et le culot obtenu est lui-même lavé superficiellement afin de compléter l'élimination des enzymes solubles. La fraction membranaire ainsi obtenue est reprise dans le tampon à 4°C de manière à obtenir une suspension comportant environ 1,5 mg de protéines par ml.

Une prise aliquote de 50 $\mu$l de la suspension membranaire est alors incubée dans un volume final de 100 $\mu$l à 25°C en présence de 10 nM de leucine-enképhaline $^3$H (39 Ci/mmole), préalablement purifiée par chromatographie sur une colonne de Porapak Q (100—120 mesh, Waters Assoc.) et de 0,1 mM de puromycine, in inhibiteur d'aminopeptidases. La durée des incubations est généralement fixée à 15 mn. de manière à mesurer la vitesse initiale de formation du tripeptide Tyr-Gly-Gly-$^3$H caractéristique de l'activité enképhaline dipeptidylcarboxypeptidasique (enképhalinasique). La réaction est arrêtée par addition de 25 $\mu$l d'HCl 0,2 N et le tripeptide est isolé par chromatographie sur colonne de Porapak Q ou sur une couche mince de silice suivant des méthodes décrites par Malfroy et al. (B. Malfroy, J. P. Swerts, C. Llorens et J. C. Schwartz, Neuro-Science Letters, *11*, 329, 1979).

Les résultats obtenus par chacune de ces deux méthodes ont toujours été en bon accord.

La mesure de la radioactivité du tripeptide est effectuée par spectrométrie en scintillation liquide.

L'effet des inhibiteurs est établi en pratiquant des expériences en présence de concentrations croissantes de ces produits, ce qui conduit à la détermination des concentrations inhibitrices 50% calculées au moyen de l'analyse des données par la méthode de Parker et Waud (J. Pharmacol. exper. Ther. *177*, 1, 1971). Dans certains cas la nature compétitive de l'inhibition a été établie par des expériences d'incubation en présence de l'inhibiteur en concentration fixe et du substrat en concentrations croissantes.

| Composés N° | Concentration inhibitrice 50% |
|---|---|
| 2 | $1,5\times10^{-6}$M |
| 5 | $2\times10^{-5}$M |
| 27 | $5\times10^{-6}$M |
| 28 | $2\times10^{-7}$M |
| 32 | $1\times10^{-7}$M |
| 35 | $4\times10^{-7}$M |
| 33 | $1\times10^{-6}$M |
| 18 | $5\times10^{-9}$M |
| 20 | $5\times10^{-9}$M |
| 22 | $7\times10^{-9}$M |
| 24 | $4\times10^{-9}$M |
| 53 | $3,8\times10^{-7}$M |
| 54 | $2,6\times10^{-7}$M |
| 55 | $3,5\times10^{-7}$M |
| 56 | $4\times10^{-7}$M |
| 57 | $7,8\times10^{-9}$M |
| 60 | $3\times10^{-8}$M |

B) Etude pharmacologique

L'étude pharmacologique des produits décrits ci-dessus a permis de mettre en évidence un effet antalgique et antidiarrhéique, un effet hypotenseur propre et une action potentialisatrice des effets d'une enképhaline, la D $Ala_2$Met Enképhaline (notamment antalgique et hypotenseur).

Les épreuves pharmacologiques réalisées ont été les suivantes:

I—Toxicité aiguë

La détermination de la mortalité chez la souris est observée à la suite de l'administration unique par voie intraveineuse de doses croissantes des composés à tester.

La $DL_{50}$, pour tous les composés étudiés, est supérieure à 100 mg/kg/i.v.

II—Toxicité subaiguë

Le composé 53 a été administré pendant 3 semaines à des souris à la dose de 50 mg/kg 3 fois/jour (150 mg par jour). Aucune modification de l'évolution pondérale et aucun signe de toxicité n'ont été présentés par les animaux par rapport aux témoins. Le poids des organes et leur examen anatomo-pathologique après sacrifice des animaux n'ont montré aucune différence par rapport aux témoins solvant.

Par ailleurs, chez les animaux chez qui on procédait à une épreuve de réversibilité à l'arrêt du traitement, aucun signe de tolérance, ni d'accoutumance et aucun phénomène de sevrage n'ont été observés.

III—Activité antalgique

1) Test de la plaque chaude:

Réflexe de lèchement de la souris sur une plaque chauffée à 55°C selon la méthode de Jacob et coll. (Arch. Int. Pharmacodyn. *122*, 287—300, 1959: *133*, 296—300, 1961).

a) Potentialisation de l'effet antalgique de la D $Ala_2$Met Enképhaline:

$\alpha$—par voie intra-ventriculaire (ivt)

Le tableau I ci-après montre que l'effet d'une dose subactive (0,3$\gamma$) de D $Ala_2$Met Enképhaline administrée par voie intra-ventriculaire est significativement potentialisée ($p<0,05$) par les composés 2, 28, 29 et 35, et que cet effet est antagonisé par la Naloxone.

$\beta$—par voie intraveineuse

Par voie intraveineuse, le n° 35 à la dose de 100 mg/kg entraîne une augmentation de 100% du temps de lèchement par rapport aux animaux traités avec la D $Ala_2$Met Enképhaline (0,3 $\gamma$) intra-ventriculaire).

TABLEAU I
Plaque chaude
Potentialisation de l'effet antalgique de la D $Ala_2$Met Enképhaline et antagonisme par la Naloxone

| Produit | Dose γ/ souris ivt | Nbre de souris | Augmentation du temps de lèchement % par rapport aux traités $DAla_2$Met enképhaline (1) |
|---|---|---|---|
| n° 2 (1) +$DAla_2$Met Enképhaline | 10 | 6 | 274* |
| n° 29 (1) +$DAla_2$Met Enképhaline | 10 | 6 | 207* |
| n° 28 (1) +$DAla_2$Met Enképhaline | 10 | 6 | 400* |
| n° 35 (1) +$DAla_2$Met Enképhaline | 10 | 10 | 240* |
| n° 35 (1) +DA $la_2$Met Enképhaline +Naloxone (10 mg/kg ss. cut.) | 10 | 10 | 18 |

(1) la dose de D $Ala_2$Met Enképhaline est de 0,3 γ/souris, dose inactive par elle-même,

* $P<0{,}05$ épreuve de rang de Wilcoxon.

b) Effet antalgique propre
Par voie intraveineuse

Le tableau ci-dessous montre que les composés 19, 53 et 54 présentent un effet antalgique dans le test de la plaque chaude à 55°C.

| Produit | $DE_{50}$ mg/kg/i.v. |
|---|---|
| 19 | 30 |
| 53 | 3 |
| 54 | 3 |

2) Test chez la souris du retrait de la queue trempée dans l'eau chauffée à 48°C selon la méthode de Sewell et Spencer (Neuropharmacology—1976—*15*, p. 683—688).

Le tableau II montre que les composés 18 et 20 administrés par voie intraveineuse à la dose de 100 mg/kg:

— présentent un effet antalgique propre,
— potentialisent très significativement la D $Ala_2$Met Enképhaline administrée 15 minutes après par voie intra-ventriculaire, à des doses subactives variant de 10 à 30γ/souris.

Cet effet est durable et supérieur à 2 heures; il est également observé à la dose de 30 mg/kg pour le n° 20.

3) Test à la Phénylbenzoquinone ou "writhing test" selon la méthode de Siegmund et coll. (Proc. Soc. Expert Biol. Med. 1957, *95*, 729—731).

Les composés 53 et 54 injectés à la dose de 0,7 mg/kg/i.v. protègent de la douleur induite par la phénylbenzoquinone les animaux traités avec une différence significative par rapport aux témoins $p<0{,}01$.

TABLEAU II
Queue de la souris
Effet antalgique propre
Potentialisation de l'effet antalgique de la D $Ala_2$Met Enképhaline

| Produits et dose | Voie | Nbre de souris | Pourcentage d'augmentation du temps de réaction | | | | |
|---|---|---|---|---|---|---|---|
| | | | $20^{mn}$ | $45^{mn}$ | $60^{mn}$ | $90^{mn}$ | $120^{mn}$ |
| N° 18—100 mg/kg | i.v. | 6 | 38 | 80* | 120* | 61* | 90 |
| N° 20—100 mg/kg | i.v. | 6 | 59 | 196* | 150* | 88* | 85 |
| N° 18—100 mg/kg +D $Ala_2$Met Enképhaline (30γivt) | i.v. | 6 | 0 | 280* | 166* | 87* | 174* |
| N° 20—100 mg/kg +D $Ala_2$Met Enképhaline (20γivt) | i.v. | 6 | 239** | 528*** | 321*** | 382*** | 322* |
| N° 20—30 mg/kg +D $Ala_2$Met Enképhaline (15γivt) | i.v. | 6 | 247** | 353*** | 283*** | 210* | 203* |

*<0,05
**<0,02
***<0,01

IV) Activité hypotensive
— propre
— potentialisatrice de l'enképhaline, (Laubie M., Schmitt H., Vincent M., Remon D. Central cardiovalscular effects of Morphinometic peptids in dogs. European Journal of Pharmacology, Vol. 46, 67—71, 1977.

Par la voie intraveineuse, les composés 18 et 24 ont entraîné une diminution de la pression artérielle dès la dose de 0,3 mg/kg.

Les résultats de ces études mettent en évidence la faible toxicité et les intéressantes propriétés inhibitrice de l'enképhalinase, antalgique et hypotensive des dérivés de l'invention qui les rendent utiles en médecine humaine et vétérinaire.

Le médicament de l'invention est administrable à l'homme par voie orale, parentérale ou rectale.

Chaque dose unitaire contient avantageusement de 0,5 à 100 mg de principe actif, les doses administrables journellement pouvant varier de 0,5 à 200 mg de principe actif.

**Revendications pour les Etats Contractants BE CH DE FR GB IT LI LU NL SE**

1. Dérivés d'amino-acides répondant à la formule générale suivante

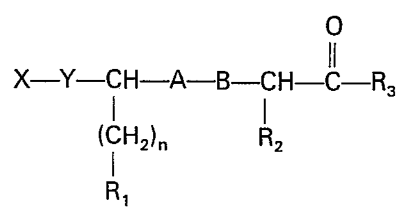

(I)

dans laquelle

*A* est un groupe carbonyle, méthylène ou amino;

*B* est un groupe amino, carbonyle ou thio;

$R_1$ est un groupe phényle éventuellement mono- ou polysubstitué par un atome d'halogène;

*n* est O ou 1;

$R_2$ est un atome d'hydrogène; un groupe alkyle en $C_1$ à $C_6$ linéaire ou ramifié; un groupe benzyle; un groupe benzyloxyalkyle;

$R_3$ est un groupe $OR_4$, $—NHR_4$ ou $N(R_4)_2$, dans lesquels $R_4$ est un atome d'hydrogène; un groupe alkyle linéaire ou ramifié en $C_1$ à $C_6$, éventuellement mono- ou polysubstitué par un atome d'halogène; un groupe phényle ou phénylalkyle($C_{1-4}$), ces deux derniers groupes étant éventuellement mono- ou polysubstitués sur le noyau phényle par un atome d'halogène; un groupe dialkyl aminoalkyle, les groupes alkyle étant en $C_1$ à $C_4$ et le groupe amino pouvant être sous forme aminoxy.

*Y* est un groupe —S—; —NH—, —$CH_2$—, ou bien Y est un groupe amino tertiaire dont la troisième valence forme avec $R_1$ un pont alcoylène à 2 atomes de carbone; et

*X* est un atome d'hydrogène; un groupe alkyle en $C_1$ ou $C_2$ substitué par un radical alcoxycarbonyle, carboxy, mercapto, acyle aliphatique ($C_{1-4}$) thio, ou benzoylthio; un groupe acyle aliphatique en $C_1$ à $C_4$ ou benzoyle; un groupe mercapto; un groupe acyle aliphatique ($C_{1-4}$) thio; un groupe benzoylthio ou un groupe phénylalkyl ($C_{1-4}$) carbonylthio éventuellement mono- ou polysubstitué par un atome d'halogène; un groupe hydroxy alkyl ($C_{1-4}$) carbonylthio ou aminoalkyl ($C_{1-4}$) carbonylthio; et leurs sels d'addition avec des acides ou des bases pharmaceutiquement acceptables, sous réserve que l'on n'ait pas simultanément X=$R_2$=H, Y=$CH_2$ et n=O et, de plus, sous réserve que dans le cas A=CO et B=NH, lorsque Y=—S—, X soit différent de H et de benzoyle et lorsque Y=—$CH_2$—, X soit différent d'un groupe alkyle en $C_1$ ou $C_2$ substitué par un radical alcoxycarbonyle ou carboxy, ou $R_2$ ne représente pas un groupe alkyle en $C_2$—$C_5$.

2. Dérivés selon la revendication 1, caractérisés en ce que:

*A* est un groupe carbonyle, méthylène ou amino;

*B* est un groupe amino, carbonyle ou thio;

$R_1$ est un groupe phényle éventuellement mono- ou polysubstitué par un atome d'halogène;

*n* est O ou 1;

$R_2$ est tel que dans la revendication 1:

$R_3$ est un groupe —$OR_4$ ou —$NHR_4$ dans lequel $R_4$ est un atome d'hydrogène; un groupe alkyle linéaire ou ramifié en $C_1$ à $C_6$ éventuellement mono- ou polysubstitué par un atome d'halogène; un groupe phénylalkyl ($C_{1-4}$) éventuellement mono- ou polysubstitué par un atome d'halogène sur le groupe phényle;

*Y* est un groupe —S—; —NH—; $CH_2$ ou bien *Y* est un groupe amino tertiaire dont la troisième valence forme avec $R_1$ un point alcoylène à 2 atomes de carbone; et

*X* est un atome d'hydrogène; un groupe alkyle en $C_1$ ou $C_2$ substitué par un radical alcoxycarbonyle, carboxy, mercapto, acyle aliphatique ($C_{1-4}$) thio, ou benzoylthio; un groupe acyle aliphatique en $C_1$ à $C_4$ ou benzoyle; un groupe mercapto; un groupe acyle aliphatique ($C_{1-4}$) thio; un groupe benzoylthio ou un groupe phénylalkyl ($C_{1-4}$) carbonylthio; et leurs sels d'addition avec des acides ou des bases pharmaceutiquement acceptables, sous réserve que l'on n'ait pas simultanément X=$R_1$=$R_2$=H, Y=$CH_2$ et n=O et, de plus, sous réserve que dans le cas A=CO et B=NH, lorsque Y=—S—, X soit différent de H et de benzoyle et lorsque Y=—$CH_2$—, X soit différent d'un groupe alkyle et $C_1$ ou $C_2$ substitué par un radical alcoxycarbonyle ou carboxy.

3. Dérivés choisis parmi les suivants:

N[(R, S) mercapto-3 benzyl-2 propionyl] glycine;

N[(R, S) mercapto-3 benzyl-2 propionyl] L-alanine;

O-benzyl, N-[(R, S) mercapto-3 benzyl-2 propionyl] L-sérine;

Ester benzylique de la N[(R, S) acétylthio-3 benzyl-2 propionyl] glycine;

Ester p.fluorobenzylique de la N[(R, S) acétylthio-3 benzyl-2 propionyl] glycine;

Ester 2,2,2-trifluoroéthylique de la N[(R, S) acétylthio-3 benzyl-2 propionyl] glycine;

Benzylamide de la N[(R, S) acétylthio-3 benzyl-2 propionyl] glycine;

Ester benzylique de la N[(R, S) mercapto-3 benzyl-2 propionyl] glycine;

Ester 2,2,2-trifluoroéthylique de la N[(R, S) mercapto-3 benzyl-2 propionyl] glycine.

4. Procédé de préparation de dérivés de formule I dans lesquels A=CO, B=NH, Y=NH, X est un groupe alkylène ou $C_1$ ou $C_2$ substitué par un groupe alcoxycarbonyle ou carboxy et $R_1$, $R_2$, $R_3$ et *n* sont tels que définis à la revendication 1, caractérisé en ce qu'on fait réagir un dérivé de formule

$$ROOC—(CH_2)_mBr \qquad (II)$$

dans laquelle R est H ou un groupe alkyle et *m* est 1 ou 2, avec un dérivé de formule

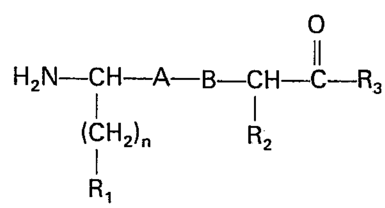

(III)

dans laquelle $R_1$, $R_2$, $R_3$ et *n* sont tels que définis ci-dessus et A=CO; B=NH.

5. Procédé de préparation de dérivés de formule I, dans lesquels A=$CH_2$, B=NH, Y=S, X est un atome d'hydrogène ou un groupe benzoyle, $R_1$ est H, *n*=O,

$R_2$ et $R_3$ sont tels que définis à la revendication 1, caractérisé en ce qu'on fait réagir

a) un thiiranne de formule $C_2H_4S$ sur un ester ou amide d'amino-acide de formule

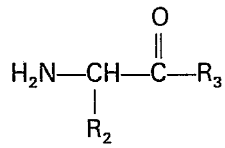

(III)

et

b) éventuellement on fait réagir le produit obtenu à l'étape (a) avec un composé de formule

R—CO—Br

dans laquelle R est un groupe alkyle inférieur ou phényle et on élimine des groupes protecteurs.

6. Procédé de préparation de dérivés de formule I, dans lesquels A=CO, B=NH, Y=NH ou $CH_2$, X est un groupe alkyle en $C_1$ ou $C_2$ substitué par un groupe mercapto acyle aliphatique thio ou benzoylthio, $R_1$, $R_2$, $R_3$ et *n* sont tels que définis à la revendication 1, caractérisé en ce qu'on réalise une réaction de condensation peptidique entre un composé de formule

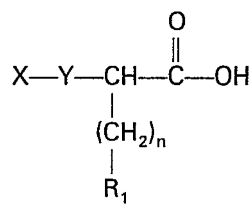

(IV)

et

un ester ou amide d'amino-acide de formule

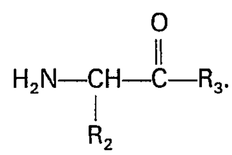

7. Procédé de préparation de dérivés de formule I, dans lesquels A=CO, B=NH, Y=S, X est un groupe acyle aliphatique en $C_1$ à $C_4$ et $R_1$, $R_2$, $R_3$ et *n* sont tels que définis à la revendication 1, caractérisé en ce qu'on fait réagir un S-acide thiocarboxylique de formule R—COSH, dans laquelle R est un radical alkyle, sur un dérivé halogéné de formule

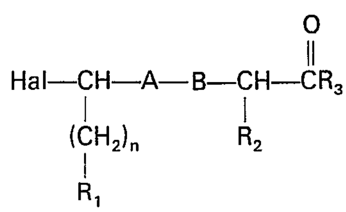

(V)

dans laquelle Hal est un atome d'halogène en particulier de brome.

8. Procédé de préparation de dérivés de formule I, dans lesquels A=CO, B=NH, Y=S, X est un groupe alkyle en $C_1$ ou $C_2$ substitué par un groupe mercapto, acyle aliphatique thio ou benzoylthio et $R_1$, $R_2$, $R_3$ et *n* sont tels que définis à la revendication 1, caractérisé en ce qu'on fait réagir un composé halogéné de formule

$$R—CO—S—(CH_2)_m—Hal$$

dans laquelle Hal est un atome d'halogène tel que le brome, R est un groupe alkyle ou phényle et *m* est 1 ou 2, avec un dérivé de formule

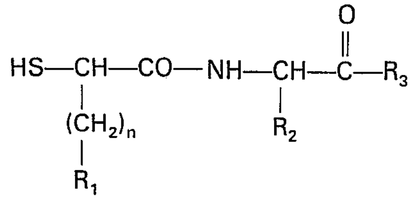

9. Procédé de préparation de dérivés de formule I, dans lesquels A=NH, B=CO, Y=NH, X est un atome d'hydrogène, un groupe alkyle substitué par un groupe carboxy ou mercapto, ou un groupe

alcoxycarbonyle et $R_1$, $R_2$, $R_3$ et *n* sont tels que définis à la revendication 1, caractérisé en ce qu'on réalise une réaction de condensation peptidique entre une diamine N-protégée de formule

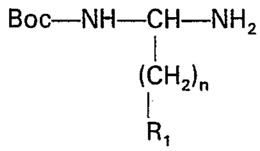

(VI)

et
un monoester ou monoamide malonique de formule

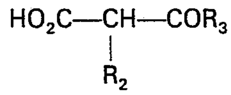

10. Procédé de préparation de dérivés de formule I, dans lesquels $A=CH_2$, B=S, Y=NH, X est un atome d'hydrogène, un groupe alkyle substitué par un groupe carboxy ou mercapto, ou un groupe alcoxycarbonyle et $R_1$, $R_2$, $R_3$ et *n* sont tels que définis à la revendication 1, caractérisé en ce qu'on réalise une réaction de condensation entre un dérivé tosylé N-protégé d'amino-acide de formule

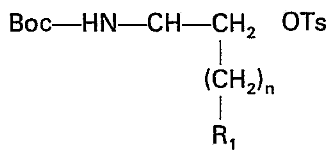

(VII)

et
un ester ou amide de l'acide thioglycolique de formule

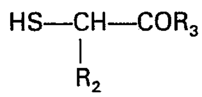

(VIII)

11. Procédé de préparation de dérivés de formule I, dans lesquels $A=—CH_2—$, B=—NH—, Y=NH, X est un atome d'hydrogène, un groupe alkyle substitué par un groupe carboxy ou mercapto, ou un groupe alcoxycarbonyle et $R_1$, $R_2$, $R_3$ et *n* sont tels que définis à la revendication 1, caractérisé en ce qu'on réalise une réaction de condensation entre un dérivé tosylé N-protégé d'amino-acide de formule

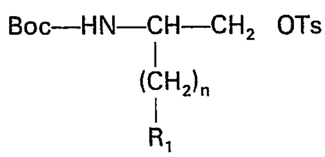

et un ester ou amide d'amino-acide de formule

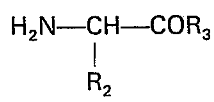

12. Médicament ayant notamment des activités inhibitrices de l'enképhalinase antaligique et hypotensive, caractérisé en ce qu'il contient à titre de principe actif un composé selon l'une quelconque des revendications 1 à 3.

13. Médicament selon la revendication 12, caractérisé en ce qu'il est présenté sous une forme appropriée pour l'administration orale, parentérale ou rectale, le principe actif étant associé à un véhicule thérapeutique convenable.

14. Médicament selon l'une quelconque des revendications 12 et 13, caractérisé en ce qu'il est présenté sous forme de doses unitaires contenant de 0,5 à 100 mg de principe actif.

**Revendications pour l'Etat Contractant AT**

1. Procédé de préparation de dérivés d'aminoacides répondant à la formule générale suivante

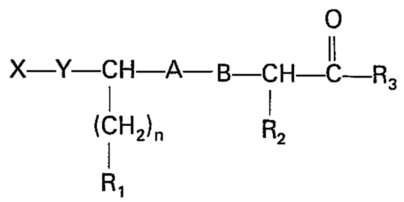

(I)

dans laquelle

*A* est un groupe carbonyle, méthylène ou amino;

*B* est un groupe amino, carbonyle ou thio;

$R_1$ est un groupe phényle éventuellement mono- ou polysubstitué par un atome d'halogène;

*n* est O ou 1;

$R_2$ est un atome d'hydrogène; un groupe alkyle en $C_1$ à $C_6$ linéaire ou ramifié; un groupe benzyle; un groupe benzyloxyalkyle;

$R_3$ est un groupe $OR_4$, $-NHR_4$ ou $N(R_4)_2$, dans lesquels $R_4$ est un atome d'hydrogène; un groupe alkyle linéaire ou ramifié en $C_1$ à $C_6$, éventuellement mono- ou polysubstitué par un atome d'halogène; un groupe phényle ou phénylalkyle $(C_{1-4})$, ces deux derniers groupes étant éventuellement mono- ou polysubstitués sur le novau phényle par un atome d'halogène; un groupe dialkyl aminoalkyle, les groupes alkyle étant en $C_1$ à $C_4$ et le groupe amino pouvant être sous forme aminoxy.

*Y* est un groupe —S—; —NH—, $-CH_2-$, ou bien Y est un groupe amino tertiaire dont la troisième valence forme avec $R_1$ un point alcoylène à 2 atomes de carbone; et

*X* est un atome d'hydrogène; un groupe alkyle en $C_1$ ou $C_2$ substitué par un radical alcoxycarbonyle, carboxy, mercapto, acyle aliphatique $(C_{1-4})$ thio, ou benzoylthio; un groupe acyle aliphatique en $C_1$ à $C_4$ ou benzoyle; un groupe mercapto; un groupe acyle aliphatique $(C_{1-4})$ thio; un groupe benzoylthio ou un groupe phénylalkyl $(C_{1-4})$ carbonylthio éventuellement mono- ou polysubstitué par un atome d'halogène; un groupe hydroxy alkyl $(C_{1-4})$ carbonylthio ou aminoalkyl $(C_{1\ 4})$ carbonylthio; et leurs sels d'addition avec des acides ou des bases pharmaceutiquement acceptables, sous réserve que l'on n'ait pas simultanément $X=R_2=H$, $Y=CH_2$ et n=O et, de plus, sous réserve que dans le cas A=CO et B=NH, lorsque Y=—S—, X soit différent de H et de benzoyle et lorsque $Y=-CH_2-$, X soit différent d'un groupe alkyle en $C_1$ ou $C_2$ substitué par un radical alcoxycarbonyle ou carboxy, caractérisé en ce que, pour préparer des dérivés de formule I dans lesquels A=CO, B=NH, Y=NH, X est un groupe alkylène en $C_1$ ou $C_2$ substitué par un groupe alcoxycarbonyle ou carboxy et $R_1$, $R_2$, $R_3$ et *n* sont tels que définis ci-dessus, on fait réagir un dérivé de formule

$$ROOC-(CH_2)_mBr \qquad (II)$$

dans laquelle R est H ou un groupe alkyle et *m* est 1 ou 2, avec un dérivé de formule

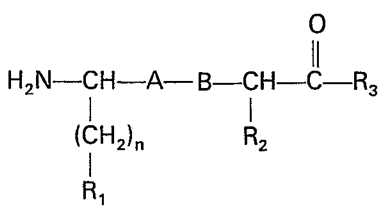

(III)

dans laquelle $R_1$, $R_2$, $R_3$ et *n* sont tels que définis ci-dessus et A=CO; B=NH, pour préparer les dérivés de formule (I), duns lesquels $A=CH_2$, B=NH, Y=S, X est un atome d'hydrogène ou un groupe benzoyle, $R_1$ est H, *n*=O, $R_2$ et $R_3$ sont tels que définis ci-dessus, on fait réagir

a) un thiiranne de formule $C_2H_4S$ sur un ester ou amide d'aminoacide de formule

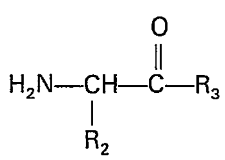

et

b) éventuellement on fait réagir le produit obtenu à l'étape (a) avec un composé de formule

$$R-CO-Br$$

dans laquelle R est un groupe alkyle inférieur ou phényle et on élimine des groupes protecteurs, pour préparer les dérivés de formule (I), dans lesquels A=CO, B=NH, Y=NH ou $CH_2$, X est un groupe alkyle en $C_1$ ou $C_2$ substitué par un groupe mercapto, acyle aliphatique thio ou benzoylthio, $R_1$, $R_2$, $R_3$ et *n* sont tels que définis ci-dessu, on réalise une réaction de condensation peptidique entre un compose de formule

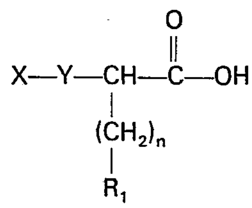

(IV)

et

un ester ou amide d'aminoacide de formule

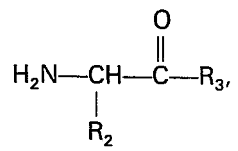

pour préparer les dérivés de formule (I), dans lesquels A=CO, B=NH, Y=S, X est un groupe acyle aliphatique en $C_1$ à $C_4$ et $R_1$, $R_2$, $R_3$ et *n* sont tels que définis ci-dessus, on fait réagir un S-acide thiocarboxylique de formule R—COSH, dans laquelle R est un radical alkyle, sur un dérivé halogéné de formule

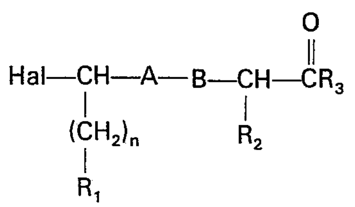

(V)

dans laquelle Hal est un atome d'halogène en particulier de brome, pour préparer les dérivés de formule (I), dans lesquels A=CO, B=NH, Y=S, X est un groupe alkyle en $C_1$ ou $C_2$ substitué par un groupe mercapto, acyle aliphatique thio ou benzoylthio et $R_1$, $R_2$, $R_3$ et *n* sont tels que définis ci-dessus, on fait réagir un composé halogéné de formule

$$R—CO—S—(CH_2)_m—Hal$$

dans laquelle Hal est un atome d'halogène tel que le brome, R est un groupe alkyle ou phényle et *m* est 1 ou 2, avec un dérivé de formule

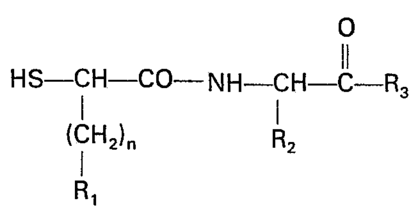

pour préparer les dérivés de formule (I), dans lesquels A=NH, B=CO, Y=NH, X est un atome d'hydrogène, un groupe alkyle substitué par un groupe carboxy ou mercapto, ou un groupe alcoxycarbonyle et $R_1$, $R_2$, $R_3$ et *n* sont tels que définis ci-dessus, on réalise une réaction de condensation peptidique entre une diamine N-protégée de formule

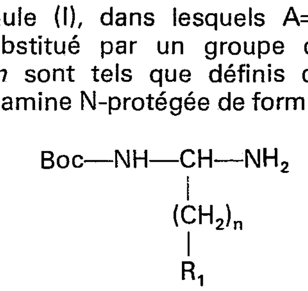

(VI)

et

un monoester ou monoamide malonique de formule

$$\begin{array}{l} HO_2C—CH—COR_3 \\ \qquad\quad | \\ \qquad\quad R_2 \end{array}$$

pour préparer les dérivés de formule (I), dans lesquels A=$CH_2$, B=S, Y=NH, X est un atome d'hydrogène, un groupe alkyle substitué par un groupe carboxy ou mercapto, ou un groupe alcoxycarbonyle et $R_1$, $R_2$, $R_3$ et *n* sont tels que définis ci-dessus, on réalise une réaction de condensation entre un dérivé tosylé N-protégé d'aminoacide de formule

$$\begin{array}{l} Boc—HN—CH—CH_2\ OTs \\ \qquad\qquad\quad | \\ \qquad\quad (CH_2)_n \\ \qquad\qquad\quad | \\ \qquad\qquad\quad R_1 \end{array} \qquad (VII)$$

et
un ester ou amide de l'acide thioglycolique de formule

$$\begin{array}{l} HS—CH—COR_3 \\ \qquad\ | \\ \qquad\ R_2 \end{array} \qquad (VIII)$$

et
pour préparer les dérivés de formule (I), dans lesquels A=—$CH_2$—, B=—NH—, Y=NH, X est un atome d'hydrogène, un groupe alkyle substitué par un groupe carboxy ou mercapto, ou un groupe alcoxycarbonyle et $R_1$, $R_2$, $R_3$ et *n* sont tels que définis ci-dessus, on réalise une réaction de condensation entre un dérivé tosylé N-protégé d'aminoacide de formule

$$\begin{array}{l} Boc—HN—CH—CH_2\ OTs \\ \qquad\qquad\quad | \\ \qquad\quad (CH_2)_n \\ \qquad\qquad\quad | \\ \qquad\qquad\quad R_1 \end{array}$$

et un ester ou amide d'aminoacide de formule

$$\begin{array}{l} H_2N—CH—COR_3 \\ \qquad\quad | \\ \qquad\quad R_2 \end{array}$$

2. Procédé selon la revendication 1, caractérisé en ce que l'on prépare les dérivés choisis parmi les suivants
N[(R, S) mercapto-3 benzyl-2 propionyl]L-leucine;
N[(R, S) mercapto-3 benzyl-2 propionyl] glycine;
N[(R, S) mercapto-3 benzyl-2 propionyl] L-alanine;
O-benzyl, N-[(R, S) mercapto-3 benzyl-2 propionyl] L-sérine;
Ester benzylique de la N[(R, S) acétylthio-3 benzyl-2 propionyl] glycine;
Ester p.fluorobenzylique de la N[(R, S) acétylthio-3 benzyl-2 propionyl] glycine;
Ester 2,2,2-trifluoroéthylique de la N[(R, S) acétylthio-3 benzyl-2 propionyl] glycine;
Benzylamide de la N[(R, S) acétylthio-3 benzyl-2 propionyl] glycine;
Ester benzylique de la N[(R, S) mercapto-3 benzyl-2 propionyl] glycine;
Ester 2,2,2-trifluoroéthylique de la N[(R, S) mercapto-3 benzyl-2 propionyl] glycine.

3. Procédé de préparation d'un médicament ayant notamment des activités inhibitrices de l'enképhalinase, antalgique et hypotensive, caractérisé en ce que l'on met un composé selon les revendications 1 ou 2 sous une forme thérapeutiquement administrable.

**Patentansprüche für die Vertragsstaaten: BE, CH, DE, FR, GB, IT, LI, LU, NL, SE**

1. Aminosäurederivate der folgenden allgemeinen Formel

$$\begin{array}{l} \qquad\qquad\qquad\qquad\qquad\quad O \\ \qquad\qquad\qquad\qquad\qquad\quad \| \\ X—Y—CH—A—B—CH—C—R_3 \\ \qquad\quad | \qquad\qquad\quad | \\ \qquad (CH_2)_n \qquad\ R_2 \\ \qquad\quad | \\ \qquad\quad R_1 \end{array} \qquad (I)$$

in der

A eine Carbonyl-, Methylen- oder Aminogruppe ist,

B eine Amino-, Carbonyl- oder Thiogruppe ist,

$R_1$ ein gegebenenfalls mit einem Halogenatom einfach oder mehrfach substituierter Phenylrest ist,

n für O oder 1 steht,

$R_2$ ein Wasserstoffatom, ein linearer oder verzweigter $C_1$—$C_6$-Alkylrest, ein Benzylrest, ein Benzyloxyalkylrest ist,

$R_3$ eine Gruppe $OR_4$, —$NHR_4$ oder $N(R_4)_2$, in der $R_4$ ein Wasserstoffatom ist, ein gegebenenfalls mit einem Halogenatom einfach oder mehrfach substituierter linearer oder verzweigter $C_1$—$C_6$-Alkylrest, ein Phenyl- oder Phenylalkylrest ($C_1$—$C_4$), wobei diese beiden letztgenannten Reste gegebenenfalls am Phenylkern mit einem Halogenatom einfach oder mehrfach substituiert sind, ein Dialkylaminoalkylrest ist, wobei die Alkylreste $C_1$— bis $C_4$-Reste sind und die Aminogruppe in Form von Aminoxy vorliegen kann,

Y eine Gruppe —S—, —NH—, —$CH_2$— ist oder Y auch eine tertiäre Aminogruppe ist, deren dritte Valenz mit $R_1$ eine Alkylenbrücke mit 2 C-Atomen bildet, und

X ein Wasserstoffatom, ein mit einem Alkoxycarbonylrest, einer Carboxylgruppe, Mercaptogruppe, aliphatischen ($C_1$—$C_4$) Acylthiogruppe oder Benzoylthiogruppe substituierter $C_1$- oder $C_2$-Alkylrest, ein aliphatischer $C_1$—$C_4$-Acylrest oder Benzoylrest, eine Mercaptogruppe, eine aliphatische ($C_1$—$C_4$) Acylthiogruppe, eine Benzoylthiogruppe oder eine gegebenenfalls mit einem Halogenatom einfach oder mehrfach substituierte Phenylalkyl ($C_1$—$C_4$)-carbonylthiogruppe, eine Hydroxyalkyl($C_1$—$C_4$)carbonylthio- oder Aminoalkyl($C_1$—$C_4$)-carbonylthiogruppe ist, und ihre Additionssalze mit pharmazeutisch annehmbaren Säuren oder Basen mit dem Vorbehalt, daß $X=R_1=R_2=H$, $Y=CH_2$ und n=O nicht gleichzeitig vorliegen und ferner unter dem Vorbehalt, daß in dem Fall A=CO und B=NH dann, wenn Y=—S— ist, X verschieden von H und Benzoyl ist und, wenn Y=—$CH_2$—, X von einem mit einer Alkoxycarbonylgruppe oder Carboxylgruppe substituierten $C_1$—$C_2$-Alkylrest verschieden ist oder $R_2$ nicht einen $C_2$—$C_5$-Alkylrest darstellt.

2. Derivate nach Anspruch 1, dadurch gekennzeichnet, daß

A eine Carbonyl-, Methylen- oder Aminogruppe ist,

B eine Amino-, Carbonyl- oder Thiogruppe ist,

$R_1$ ein gegebenenfalls mit einem Halogenatom einfach oder mehrfach substituierter Phenylrest ist,

n für O oder 1 steht,

$R_2$ die in Anspruch 1 genannte Bedeutung hat,

$R_3$ eine Gruppe —$OR_4$ oder —$NHR_4$, in der $R_4$ ein Wasserstoffatom ist, ein gegebenenfalls mit einem Halogenatom einfach oder mehrfach substituierter linearer oder verzweigter $C_1$—$C_8$-Alkylrest, ein gegebenenfalls mit einem Halogenatom am Phenylrest einfach oder mehrfach substituierter Phenylalkyl($C_1$—$C_4$)-rest ist,

Y eine Gruppe —S—, —NH—, $CH_2$ oder Y auch eine tertiäre Aminogruppe ist, deren dritte Valenz mit $R_1$ eine Alkylenbrücke mit 2 C-Atomen bildet, und

X ein Wasserstoffatom, ein mit einer Alkoxycarbonylgruppe, Carboxylgruppe, Mercaptogruppe, aliphatischen $C_1$—$C_4$-Acylgruppe oder Benzoylthiogruppe substituierter $C_1$- oder $C_2$-Alkylrest, eine aliphatische $C_1$—$C_4$-Acylgruppe oder Benzoylgruppe, eine Mercaptogruppe, eine aliphatische ($C_1$—$C_4$) Acylthiogruppe, eine Benzoylthio- oder eine Phenylalkyl($C_1$—$C_4$)-carbonylthiogruppe ist, und ihre Additionssalze mit pharmazeutisch annehmbaren Säuren oder Basen, mit dem Vorbehalt, daß $X=R_1=R_2=H$, $Y=CH_2$ und n=O nicht gleichzeitig vorliegen, und ferner unter dem Vorbehalt, daß in dem Falle A=CO und B=NH dann, wenn X=—S—, X verschieden von H und Benzoyl ist und dann, wenn Y=—$CH_2$—, X von einem mit einer Alkoxycarbonyl- oder Carboxylgruppe substituierten $C_1$- oder $C_2$-Alkylrest verschieden ist.

3. Derivate, ausgewählt unter den folgenden:

N-[(R, S)-3-Mercapto-2-benzylpropionyl]L-leucin,

N[(R, S)-3-Mercapto-2-benzylpropionyl]glycin,

N[(R, S)-3-Mercapto-2-benzylpropionyl]L-alanin,

O-Benzyl, N-[(R, S)-3-mercapto-2-benzylpropionyl]-L-serin,

Benzylester von N[(R, S)-3-Acetylthio-2-benzylpropionyl]glycin,

p-Fluorobenzylester von N[(R, S)-3-Acetylthio-2-benzylpropionyl]glycin.

2,2,2-Trifluoroethylester von N[(R, S)-3-Acetylthio-2-benzylpropionyl]glycin,

Benzylamid von N[(R, S)-3-Acetylthio-2-benzylpropionyl]glycin,

Benzylester von N[(R, S)-3-Mercapto-2-benzylpropionyl]glycin,

2,2,2-Trifluoroethylester von N[(R, S)-3-Mercapto-2-benzylpropionyl]glycin.

4. Verfahren zur Herstellung von Derivaten der Formel I, in denen A=CO, B=NH, Y=NH, X ein mit einer Alkoxycarbonyl- oder Carboxylgruppe substituierter $C_1$- oder $C_2$-Alkylenrest ist und $R_1$, $R_2$, $R_3$ und n die in Anspruch 1 genannten Bedeutungen haben, dadurch gekennzeichnet, daß man ein Derivat der Formel

$$ROOC—(CH_2)_mBr \qquad (II)$$

worin R für H oder einen Alkylrest und m für 1 oder 2 steht, mit einem Derivat der Formel

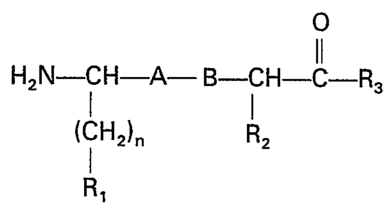

(III)

in der $R_1$, $R_2$, $R_3$ und *n* die vorstehend genannten Bedeutungen haben und A=CO und B=NH, umsetzt.

5. Verfahren zur Herstellung von Derivaten der Formel I, in denen $A=CH_2$, B=NH, Y=S, X ein Wasserstoffatom oder ein Benzoylrest ist, $R_1$ für H, n für O steht, $R_2$ und $R_3$ die in Anspruch 1 genannten Bedeutungen haben, dadurch gekennzeichnet, daß man

a) ein Thiiran der Formel $C_2H_4S$ mit einem Aminosäureester oder -amid der Formel

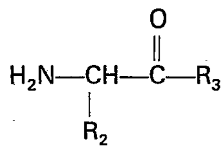

(III)

umsetzt und

b) das in der Stufe (a) erhaltene Produkt gegebenenfalls mit einer Verbindung der Formel

R—CO—Br,

in der R ein niederer Alkylrest oder ein Phenylrest ist, umsetzt und die Schutzgruppen entfernt.

6. Verfahren zur Herstellung von Derivaten der Formel I, in denen A=CO, B=NH, Y=NH oder $CH_2$, X ein mit einer aliphatischen Mercaptoacylthio- oder Benzoylthiogruppe substituierter $C_1$- oder $C_2$-Alkylrest ist, $R_1$, $R_2$, $R_3$ und n die in Anspruch 1 genannten Bedeutungen haben, dadurch gekennzeichnet, daß man eine Peptidkondensationsreaktion zwischen einer Verbindung der Formel

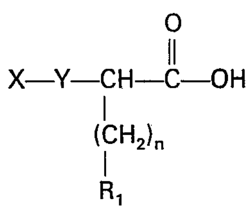

(IV)

und einem Aminosäureester oder -amid der Formel

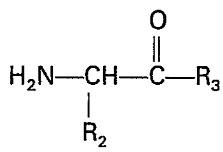

durchführt.

7. Verfahren zur Herstellung von Derivaten der Formel I, in denen A=CO, B=NH, Y=S, X ein aliphatischer $C_1$—$C_4$-Acylrest ist und $R_1$, $R_2$, $R_3$ und n die in Anspruch 1 genannten Bedeutungen haben, dadurch gekennzeichnet, daß man eine Thiocarbon(S)-säure der Formel R—COSH, in der R ein Alkylrest ist, mit einem halogenierten Derivat der Formel

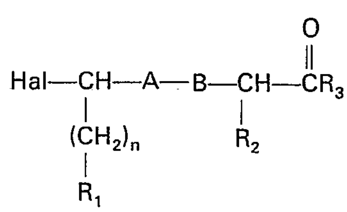

(V)

in der Hal ein Halogenatom, insbesondere Brom ist, umsetzt.

8. Verfahren zur Herstellung von Derivaten der Formel I, in denen A=CO, B=NH, Y=S, X ein mit einer Mercaptogruppe, eine aliphatischen Thioacylgruppe oder Benzoylthiogruppe substituierter $C_1$- oder $C_2$-Alkylrest ist und $R_1$, $R_2$, $R_3$ und n die in Anspruch 1 genannten Bedeutungen haben, dadurch gekennzeichnet, daß man eine halogenierte Verbindung der Formel

$$R—CO—S—(CH_2)_m—Hal$$

in der Hal ein Halogenatom, z.B. Brom ist, R ein Alkyl- oder Phenylrest ist und m für 1 oder 2 steht, mit einem Derivat der Formel

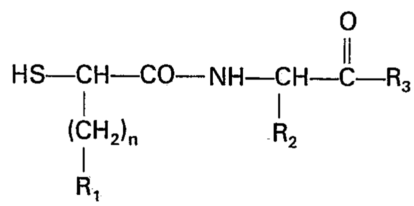

umsetzt.

9. Verfahren zur Herstellung von Derivaten der Formel I, in denen A=NH, B=CO, Y=NH, X ein Wasserstoffatom, ein mit einer Carboxyl- oder Mercaptogruppe substituierter Alkylrest oder eine Alkoxycarbonylgruppe ist und $R_1$, $R_2$, $R_3$ und n die in Anspruch 1 genannten Bedeutungen haben, dadurch gekennzeichnet, daß man eine Peptidkondensationsreaktion zwischen einem N-geschützten Diamin der Formel

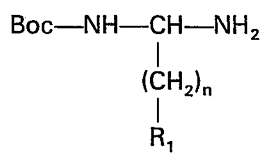

(VI)

und einem Malonsäuremonoester oder -monoamid der Formel

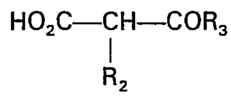

durchführt.

10. Verfahren zur Herstellung von Derivaten der Formel I, in denen $A=CH_2$, B=S, Y=NH, X ein Wasserstoffatom, ein mit einer Carboxyl- oder Mercaptogruppe substituierter Alkylrest oder eine Alkoxycarbonylgruppe ist und $R_1$, $R_2$, $R_3$ und n die in Anspruch 1 genannten Bedeutungen haben, dadurch gekennzeichnet, daß man eine Kondensationsreaktion zwischen einem N-geschützten Aminosäuretosylderivat der Formel

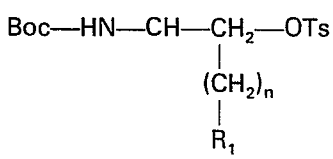

(VII)

und einem Thioglycolsäureester oder -amid der Formel

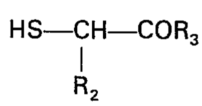

(VIII)

durchführt.

11. Verfahren zur Herstellung von Derivaten der Formel I, in denen $A=—CH_2—$, $B=—NH—$, Y=NH, X ein Wasserstoffatom, ein mit einer Carboxyl- oder Mercaptogruppe substituierter Alkylrest oder eine Alkoxycarbonylgruppe ist und $R_1$, $R_2$, $R_3$ und n die in Anspruch 1 genannten Bedeutungen haben, dadurch gekennzeichnet, daß man eine Kondensationsreaktion zwischen einem N-geschützten Aminosäuretosylderivat der Formel

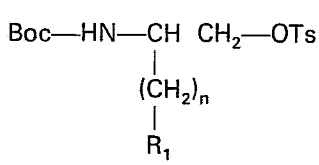

un einem Aminosäureester oder -amid der Formel

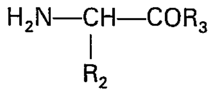

durchführt.

12. Medikament mit insbesondere hemmenden Wirksamkeiten gegen die analgetische und hypotensive Enzephalinase, dadurch gekennzeichnet, daß es als Wirkstoff eine Verbindung nach irgendeinem der Ansprüche 1 bis 3 enthält.

13. Medikament nach Anspruch 12, dadurch gekennzeichnet, daß es in einer für die orale, parenterale oder rektale Verabreichung geeigneten Form dargeboten wird, wobei der Wirkstoff mit einem geeigneten therapeutischen Träger kombiniert ist,

14. Medikament nach irgendeinem der Ansprüche 12 und 13, dadurch gekennzeichnet, daß es in Form von Einheitsdosen, die 0,5 bis 100 mg Wirkstoff enthalten, dargeboten wird.

**Patentansprüche für den Vertragsstaat: AT**

1. Verfahren zur Herstellung von Aminosäurederivaten der folgenden allgemeinen Formel

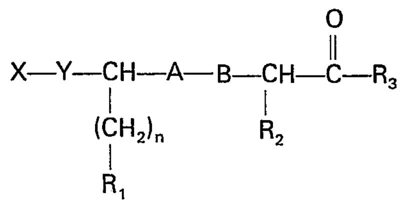

(I)

in der

A eine Carbonyl-, Methylen- oder Aminogruppe ist,

B eine Amino-, Carbonyl- oder Thiogruppe ist,

$R_1$ ein gegebenenfalls mit einem Halogenatom einfach oder mehrfach substituierter Phenylrest ist, n für O oder 1 steht,

$R_2$ ein Wasserstoffatom, ein linearer oder verzweigter $C_1$—$C_6$-Alkylrest, ein Benzylrest, ein Benzyloxyalkylrest ist,

$R_3$ ein Gruppe $OR_4$, —$NHR_4$ oder $N(R_4)_2$, in der $R_4$ ein Wasserstoffatom ist, ein gegebenenfalls mit einem Halogenatom einfach oder mehrfach substituierter linearer oder verzweigter $C_1$—$C_6$-Alkylrest, ein Phenyl- oder Phenylalkylrest ($C_1$—$C_4$), wobei diese beiden letztgenannten Reste gegebenenfalls am Phenylkern mit einem Halogenatom einfach oder mehrfach substituiert sind, ein Dialkylaminoalkylrest ist, wobei die Alkylreste $C_1$- bis $C_4$-Reste sind und die Aminogruppe in Form von Aminoxy vorliegen kann,

Y eine Gruppe —S—, —NH—, —$CH_2$— ist oder Y auch eine tertiäre Aminogruppe ist, deren dritte Valenz mit $R_1$ eine Alkylenbrücke mit 2 C-Atomen bildet, und

X ein Wasserstoffatom, ein mit einem Alkoxycarbonylrest, einer Carboxylgruppe, Mercaptogruppe, aliphatischen ($C_1$—$C_4$) Acylthiogruppe oder Benzoylthiogruppe substituierter $C_1$- oder $C_2$-Alkylrest, ein aliphatischer $C_1$—$C_4$-Acylrest oder Benzoylrest, eine Mercaptogruppe, eine aliphatische ($C_1$—$C_4$) Acylthiogruppe, eine Benzoylthiogruppe oder eine gegebenenfalls mit einem Halogenatom einfach oder mehrfach substituierte Phenylalkyl($C_1$—$C_4$)-carbonylthiogruppe, eine Hydroxyalkyl($C_1$—$C_4$)carbonylthio- oder Aminoalkyl($C_1$—$C_4$)carbonylthiogruppe ist, und ihrer Additionssalze mit pharmazeutisch annehmbaren Säuren oder Basen mit dem Vorbehalt, daß $X=R_1=R_2=H$, $Y=CH_2$ und n=O nicht gleichzeitig vorliegen und ferner unter dem Vorbehalt, daß in dem Fall A=CO und B=NH dann, wenn Y=—S— ist, X verschieden von H und Benzoyl ist und, wenn Y=—$CH_2$—, X von einem mit einer Alkoxycarbonylgruppe oder Carboxylgruppe substituierten $C_1$- oder $C_2$-Alkylrest verschieden ist, dadurch gekennzeichnet, daß man zur Herstellung der Derivate der Formel I, in denen A=CO, B=NH, Y=NH, X ein mit einer Alkoxycarbonyl- oder Carboxylgruppe substituierter $C_1$- oder $C_2$-Alkylenrest ist und $R_1$, $R_2$, $R_3$ und n die vorstehend genannten Bedeutungen haben, ein Derivat der Formel

$$ROOC—(CH_2)_mBr \qquad (II)$$

in der R für H oder einen Alkylrest und m für 1 oder 2 steht, mit einem Derivat der Formel

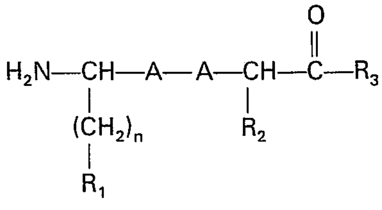

(III)

in der $R_1$, $R_2$, $R_3$ und n die vorstehend genannten Bedeutungen haben und A=CO und B=NH, umsetzt;

man zur Herstellung der Derivate der Formel (I), in denen A=$CH_2$, B=NH, Y=S, X ein Wasserstoffatom oder eine Benzoylgruppe ist,

$R_1$ für H steht, n=O ist, $R_2$ und $R_3$ die vorstehend genannten Bedeutungen haben,

a) ein Thiiran der Formel $C_2H_4S$ mit einem Aminosäureester oder -amid oder Formel

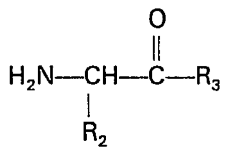

umsetzt und

b) das in der Stufe (a) erhaltene Produkt gegebenenfalls mit einer Verbindung der Formel

$$R—CO—Br$$

in der R ein niederer Alkylrest oder ein Phenylrest ist, umsetzt und die Schutzgruppen entfernt; man zur Herstellung der Derivate der Formel (I), in denen A=CO, B=NH, Y=NH oder $CH_2$, X ein mit einer Mercaptogruppe, einer aliphatischen Acylthio- oder Benzoylthiogruppe substituierter $C_1$- oder $C_2$-Alkylrest ist, $R_1$, $R_2$, $R_3$ und n die vorstehend genannten Bedeutungen haben, eine Peptidkondensationsreaktion zwischen einer Verbindung der Formel

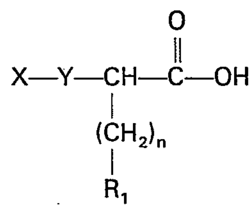 (IV)

und einem Aminosäureester oder -amid der Formel

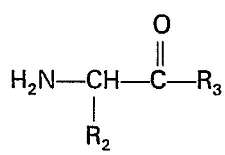

durchführt;

man zur Herstellung der Derivate der Formel (I), in denen A=CO, B=NH, Y=S, X eine aliphatische $C_1$—$C_4$-Acylgruppe ist und $R_1$, $R_2$, $R_3$ und n die vorstehend genannten Bedeutungen haben, eine Thiocarbon(S)-säure der Formel R—COSH, in der R ein Alkylrest ist, mit einem halogenierten Derivat der Formel

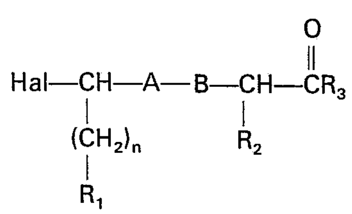 (V)

in der Hal ein Halogenatom, insbesondere Brom ist, umsetzt;

man zur Herstellung der Derivate der Formel (I), in denen A=CO, B=NH, Y=S, X ein mit einer Mercaptogruppe, einer aliphatischen Thioacylgruppe oder Benzoylthiogruppe substituierter $C_1$- oder $C_2$-Alkylrest ist und $R_1$, $R_2$, $R_3$ und n die vorstehend genannten Bedeutungen haben, mit einer halogenierten Verbindung der Formel

$$R—CO—S—(CH_2)_m—Hal,$$

in der Hal ein Halogenatom, z.B. Brom ist, R ein Alkyl- oder Phenylrest ist und m für 1 oder 2 steht, mit einem Derivat der Formel

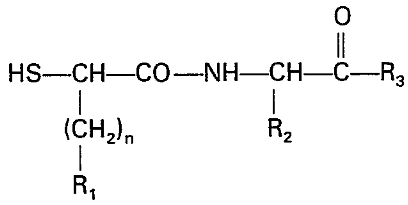

umsetzt;

man zur Herstellung der Derivate der Formel (I), in denen A=NH, B=CO, Y=NH, X ein Wasserstoffatom, ein mit einer Carboxyl- oder Mercaptogruppe substituierter Alkylrest oder eine Alkoxycarbonylgruppe ist und $R_1$, $R_2$, $R_3$ und n die vorstehend genannten Bedeutungen haben, eine Peptidkondensationsreaktion zwischen einem N-geschützten Diamin der Formel

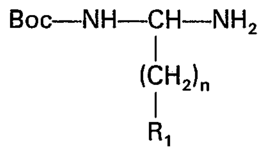

(VI)

und einem Malonsäuremonoester oder -monoamid der Formel

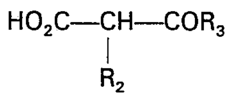

durchführt;

man zur Herstellung der Derivate der Formel (I), in denen A=$CH_2$, B=S, Y=NH, X ein Wasserstoffatom, ein mit einer Carboxyl- oder Mercaptogruppe substituierter Alkylrest oder eine Alkoxycarbonylgruppe ist und $R_1$, $R_2$, $R_3$ und n die vorstehend genannten Bedeutungen haben, eine Kondensationsreaktion zwischen einem N-geschützten Aminosäuretosylderivat der Formel

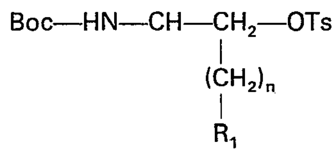

(VII)

und einem Thioglycolsäureester oder -amid der Formel

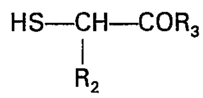

(VIII)

durchführt und

man zur Herstellung der Derivate der Formel (I), in den A=—$CH_2$—, B=—NH—, Y=NH, X ein Wasserstoffatom, ein mit einer Carboxyl- oder Mercaptogruppe substituierter Alkylrest oder eine Alkoxycarbonylgruppe ist und $R_1$, $R_2$, $R_3$ und n die vorstehend genannten Bedeutungen haben, eine Kondensationsreaktion zwischen einem N-geschützten Aminosäuretosylderivat der Formel

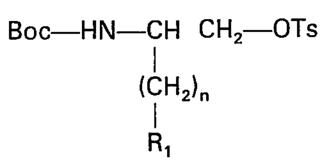

und einem Aminosäureester oder -amid der Formel

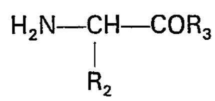

durchführt.

2. Verfahren nach Anspruch 1, dadurch gekennzeichnet, daß man die Derivate herstellt, die unter den folgenden ausgewählt sind:

N[(R, S)-3-Mercapto-2-benzylpropionyl]L-leucin,
N[(R, S)-3-Mercapto-2-benzylpropionyl]glycin,
N[(R, S)-3-Mercapto-2-benzylpropionyl]L-alanin,
O-Benzyl,N-[(R, S)-3-mercapto-2-benzylpropionyl]-L-serin,
Benzylester von N[(R, S)-3-Acetylthio-2-benzylpropionyl]glycin,
p-Fluorobenzylester von N[(R, S)-3-Acetylthio-2-benzylpropionyl]glycin,
2,2,2-Trifluoroethylester von N[(R, S)-3-Acetylthio-2-benzylpropionyl]glycin,
Benzylamid von N[(R, S)-3-Acetylthio-2-benzylpropionyl]glycin,
Benzylester von N[(R, S)-3-Mercapto-2-benzylpropionyl]glycin,
2,2,2-Trifluoroethylester von N[(R, S)-3-Mercapto-2-benzylpropionyl]glycin.

3. Verfahren zur Herstellung eines Medikaments mit insbesondere hemmenden Wirksamkeiten gegen analgetische und hypotensive Enzephalinase, dadurch gekennzeichnet, daß man ein Verbindung nach den Ansprüchen 1 oder 2 in eine therapeutisch verabreichbare Form bringt.

**Claims for the Contracting States: BE, CH, DE, FR, GB, IT, LI, LU, NL, SE**

1. Aminoacid derivatives having the following general formula:

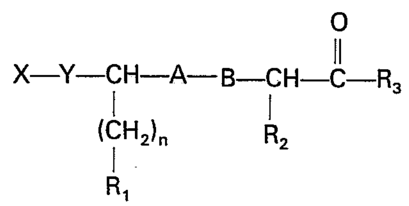

(I)

in which:

A is a carbonyl, methylene, or amino group;

B is an amino, a carbonyl or a thio group;

$R_1$ is a phenyl group, optionally monosubstituted or polysubstituted with a halogen atom;

*n* is O or 1;

$R_2$ is a hydrogen; straight-chain or branched-chain $C_{1-6}$ alkyl group; a benzyl group; a benzyloxy-alkyl group;

$R_3$ is an —$OR_4$ or —$NHR_4$ group in which $R_4$ is a hydrogen atom; a straight- or branched $C_{1-6}$ alkyl, chain alkyl group, optionally mono- or poly-halosubstituted; a phenyl or phenyl($C_{1-4}$)alkyl optionally mono- or polyhalosubstituted on the phenyl nucleus; a dialkylaminoalkyl group in which the alkyl groups contain 1—4 carbon atoms and the amino group may be in aminoxy form;

*Y* is an —S—, —NH—, —$CH_2$— group or Y is a tertiary amino group whose third valency forms with $R_1$ an alkylene bridge having 2 carbon atoms; and

*X* is a hydrogen atom; a $C_{1-2}$ alkyl substituted with alkoxycarbonyl, carboxy, mercapto, aliphatic $C_{1-4}$acyl thio, or benzylthio, a $C_{1-4}$aliphatic acyl or benzoyl; mercapto; aliphatic $C_{1-4}$acyl thio; benzoylthio or phenyl($C_{1-4}$alkyl) carbonylthio, optionally mono- or polyhalosubstituted; hydroxy($C_{1-4}$alkyl)carbonylthio; or amino($C_{1-4}$alkyl)carbonylthio; and their addition salts with pharmaceutically acceptable acids and bases, provided that there are not simultaneously X=$R_2$=H, Y=$CH_2$ and *n*=O, and further provided that when A=CO and B=NH, when Y=—S—, X is different from H and benzoyl, and when Y=—$SH_2$—, X is different from a $C_{1-2}$ alkyl substituted with alcoxycarbonyl or carboxy, or $R_2$ is not a $C_{2-5}$ alkyl group.

2. Derivatives as claimed in claim 1, wherein:

*A* is a carbonyl, methylene or amino group;

*B* is an amino, carbonyl or thio group;

$R_1$ is a phenyl optionally mono- or polyhalosubstituted;

*n* is O or 1;

$R_2$ is as in claim 1;

$R_3$ is an —$OR_4$ or —$NHR_4$ group in which $R_4$ is a hydrogen atom; a straight- or branched $C_{1-6}$ alkyl, optionally mono- or poly-halosubstituted; a phenyl($C_{1-4}$alkyl) optionally mono- or poly-halosubstituted on the phenyl nucleus;

*Y* is —S—; —NH—, —$CH_2$—, or Y is a tertiary amino in which the third valency forms with $R_1$ an alkylene bridge containing 2 carbon atoms; and

*X* is selected from: hydrogen; $C_{1-2}$ alkyl substituted with a substituent selected from alkoxycarbonyl, carboxy, mercapto, aliphatic $C_{1-4}$ acylthio, and benzoylthio; aliphatic $C_{1-4}$ acyl; benzoyl; mercapto; aliphatic $C_{1-4}$ acylthio; benzoylthio; phenyl($C_{1-4}$alkyl)carbonylthio; and their addition salts with pharmaceutically acceptable acids and bases, provided that there are not simultaneously X=$R_1$=$R_2$=H, Y=$CH_2$ and *n*=O, and further provided that when A=CO and B=NH, when Y=—S—, X is different from H and benzoyl, and when Y=—$CH_2$—, X is different from a $C_{1-2}$ alkyl substituted with alcoxycarbonyl or carboxy.

3. Derivatives selected from the following:
N-[(R, S)-3-mercapto-2-benzyl-propionyl]-glycine;
N-[(R, S)-3-mercapto-2-benzyl-propionyl]-L-alanine;
O-benzyl,N-[(R, S)-3-mercapto-2-benzyl-propionyl]-L-serine;
N-[(R, S)-3-acetylthio-2-benzyl-propionyl]-glycine benzyl ester;
N-[(R, S)-3-acetylthio-2-benzyl-propionyl]glycine p.fluorobenzyl ester;
N-[(R, S)-3-acetylthio-2-benzyl-propionyl]glycine 2,2,2-trifluoroethyl ester;
N-[(R, S)-3-acetylthio-2-benzyl-propionyl]glycine benzylamide;
N-[(R, S)-3-mercapto-2-benzyl-propionyl]glycine benzyl ester; and
N-[(R, S)-3-mercapto-2-benzyl-propionyl]glycine 2,2,2-trifluoroethyl ester.

4. Process for the preparation of derivatives of the formula (I) in which: $A$=CO, $B$=NH, $Y$=NH, $X$ is $C_{1-2}$ alkylene substituted with a substituent selected from alkoxycarbonyl and carboxy, and $R_1$, $R_2$, $R_3$ and $n$ are as defined in claim 1, comprising reacting a derivative of the formula:

$$ROOC—(CH_2)_mBr \qquad (II)$$

in which R is H or an alkyl group, and $m$ is 1 or 2, with a derivative of the formula:

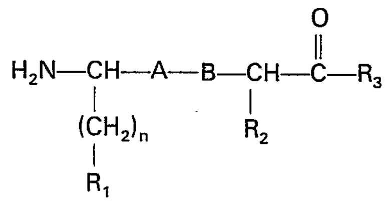

(III)

in which $R_1$, $R_2$, $R_3$ and $n$ have the above-defined meanings and $A$=CO, $B$=NH.

5. Process for the preparation of derivatives of the formula (I) in which: $A$=—$CH_2$—, $B$=—NH, $Y$=S, $X$ is a hydrogen atom or a benzoyl group, $R_1$=H, $n$=O, $R_2$ and $R_3$ are as defined in claim 1, comprising reacting:

a) a thiiran of the formula $C_2H_4S$ with a compound selected from aminoacid ester and amide of the formula

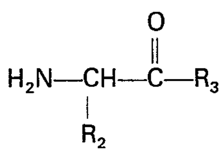

and

b) if desired, reacting the product of step (a) with a compound of the formula:

$$R—CO—Br$$

in which R is a lower alkyl or phenyl, and removing protecting groups.

6. Process for the preparation of derivatives of the formula (I), in which: $A$=CO, $B$=NH, $Y$ is selected from NH and $CH_2$, $X$ is $C_{1-2}$ alkyl substituted with mercapto, aliphatic acylthio, or benzoylthio, $R_1$, $R_2$, $R_3$ and $n$ are as defined in claim 1, comprising carrying out a peptidic condensation reaction between a compound of the formula:

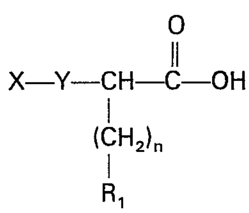

(IV)

and an aminoacid ester and amide of the formula:

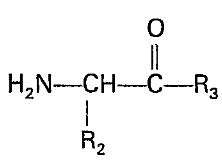

7. Process for the preparation of derivatives of the formula (I), in which: $A$=CO, $B$=NH, $Y$=S, $X$ is an aliphatic $C_{1-4}$ acyl, and $R_1$, $R_2$, $R_3$ and $n$ are as defined in claim 1, comprising reacting a

thiocarboxylic S-acid of the formula R—COSH, in which R is an alkyl group, with a halogenated derivative of the formula:

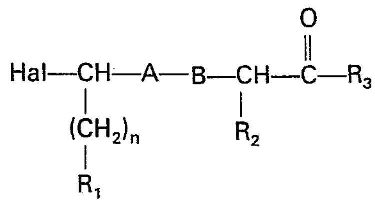 (V)

in which Hal is halogen, particularly bromine.

8. Process for the preparation of derivatives of the formula (I), in which: *A*=CO, *B*=NH, *Y*=S, *X* is $C_{1-2}$ alkyl substituted with mercapto, aliphatic acylthio, or benzoylthio and $R_1$, $R_2$, $R_3$ and *n* are as defined in claim 1, comprising reacting a halogenated compound of the formula:

$$R—CO—S—(CH_2)_m—Hal$$

in which Hal is halogen such as bromine, R is an alkyl or phenyl group and *m* is 1 or 2, with a derivative having the formula:

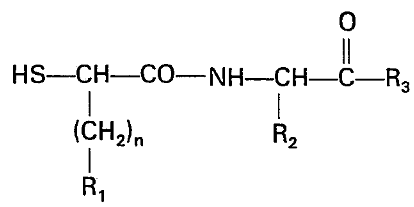

9. Process for the preparation of derivatives of the formula (I), in which: *A*=NH, *B*=CO, *Y*=NH, *X* is a hydrogen, an alkyl substituted with a carboxy or mercapto, or an alkoxycarbonyl group and $R_1$, $R_2$, $R_3$ and *n* are as defined in claim 1, comprising carrying out a peptidic condensation reaction between a N-protected diamine having the formula:

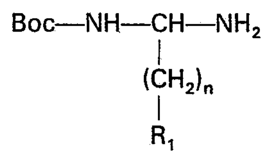 (VI)

and a malonic monoester or monoamide of the formula:

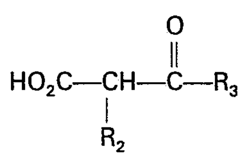

10. Process for the preparation of derivatives of the formula (I), in which: $A$=$CH_2$, *B*=S, *Y*=NH, *X* is a hydrogen, an alkyl substituted with a carboxy or mercapto or an alkoxycarbonyl, and $R_1$, $R_2$, $R_3$ and *n* are as defined in claim 1, comprising carrying out a condensation reaction between a N-protected tosylated aminoacid derivative having the formula:

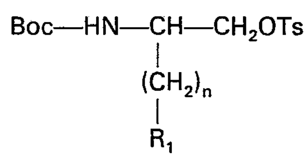 (VII)

and a thioglycolic acid ester or amide having the formula:

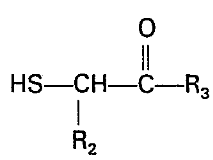 (VIII)

11. Process for the preparation of derivatives of the formula (I), in which: $A$=—$CH_2$, $B$=—NH—, $Y$=NH, $X$ is an alkyl substituted with a carboxy or mercapto group, or an alkoxycarbonyl, and $R_1$, $R_2$ and $R_3$ are as defined in claim 1, comprising effecting a condensation reaction between a N-protected tosylated aminoacid derivative having the formula:

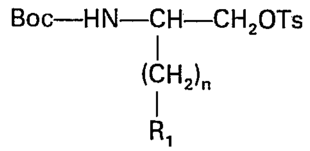

and an aminoacid ester or amide of the formula:

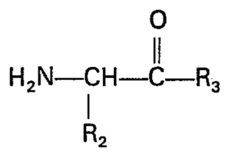

12. A medicament having particularly enkephalinase-inhibiting, antalgic, and hypotensive activities, comprising, as active ingredient a compound according to any one of the claims 1 to 3.

13. A medicament according to claim 12, presented in a form suitable for oral, parenteral or rectal administration, the active principle being associated to a therapeutically acceptable carrier.

14. A medicament as claimed in claims 12 and 13, presented in unit dosage form, each unit dose containing 0.5—100 mg active ingredient.

**Claims for the Contracting State: AT**

1. Process for the preparation of aminoacid derivatives having the following general formula:

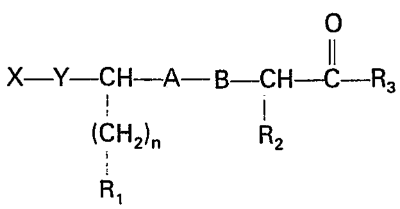

(I)

in which:

A is a carbonyl, methylene, or amino group;

B is an amino, a carbonyl or a thio group;

$R_1$ is a phenyl group, optionally monosubstituted or polysubstituted with a halogen atom;

$n$ is 0 or 1;

$R_2$ is a hydrogen; straight-chain or branched-chain $C_{1-6}$ alkyl group; a benzyl group; a benzyloxylalkyl group;

$R_3$ is an $OR_4$, $NHR_4$ or $N(R_4)_2$ group in which $R_4$ is a hydrogen atom; a $C_{1-6}$ straight) and branched chain alkyl group, optionally mono- or poly-halo-substituted; phenyl or phenyl($C_{1-4}$)alkyl optionally mono- or polyhalosubstituted on the phenyl nucleus; a dialkylaminoalkyl group in which the alkyl groups contain 1—4 carbon atoms and the amino group may be in aminoxy form;

$Y$ is an —S—, —NH—, —$CH_2$— group or Y is a tertiary amino group whose third valency forms with $R_1$ an alkylene bridge having 2 carbon atoms; and

$X$ is a hydrogen atom; a $C_{1-2}$ alkyl substituted with alkoxycarbonyl, carboxy, mercapto, aliphatic $C_{1-4}$ acyl thio, or benzylthio, a $C_{1-4}$ aliphatic acyl or benzoyl; mercapto; aliphatic $C_{1-4}$acyl thio; benzoylthio or phenyl ($C_{1-4}$alkyl) carbonylthio, optionally mono- or polyhalosubstituted; hydroxy($C_{1-4}$alkyl)carbonylthio; or amino($C_{1-4}$alkyl)carbonylthio; and their addition salts with pharmaceutically acceptable acids and bases, provided that there are not simultaneously X=$R_2$=H, Y=$CH_2$ and $n$=O, and further provided that when A=CO and B=NH, when Y=—S—, X is different from H and benzoyl, and when Y=—$SH_2$—, X is different from a $C_{1-2}$ alkyl substituted with alcoxycarbonyl or carboxy, characterized in that for the preparation of derivatives of the formula (I), in which: $A$=CO, $B$=NH, $Y$=NH, $X$ is $C_{1-2}$ alkylene substituted with a substituent selected from alkoxycarbonyl and carboxy, and $R_1$, $R_2$, $R_3$ and $n$ are as defined above, a derivative of the formula:

$$ROOC—(CH_2)_mBr \qquad (II)$$

in which R is H or an alkyl group, and $m$ is 1 or 2, is reacted with a derivative of the formula:

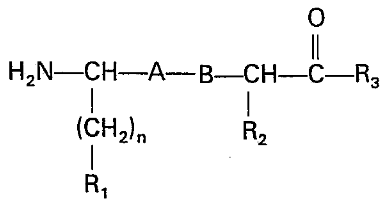

(III)

in which $R_1$, $R_2$, $R_3$ and *n* have the above-defined meanings and *A*=CO, *B*=NH, for the preparation of derivatives of the formula (I), in which: *A*=—$CH_2$—, *B*=—NH, *Y*=S, *X* is a hydrogen atom or a benzoyl group, $R_1$=H, *n*=O, $R_2$ and $R_3$ are as defined above:

a) a thiiran of the formula $C_2H_4S$ is reacted with a compound selected from aminoacid ester and amide of the formula

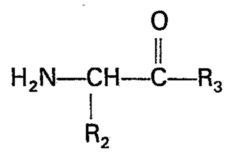 and

b) if desired, the product of step (a) is reacted with a compound of the formula:

R—CO—Br

in which R is a lower alkyl or phenyl, and the protecting groups are removed, for the preparation of derivatives of the formula (I), in which: *A*=CO, *B*=NH, *Y* is selected from NH and $CH_2$, *X* is $C_{1-2}$ alkyl substituted with mercapto, aliphatic acylthio, or benzoylthio, $R_1$, $R_2$, $R_3$ and *n* are as defined above, a peptidic condensation reaction is carried out between a compound of the formula:

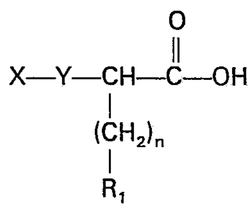

(IV)

and an aminoacid ester and amide of the formula:

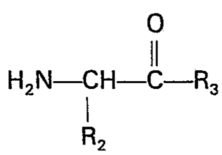

for the preparation of derivatives of the formula (I), in which: *A*=CO, *B*=NH, *Y*=S, *X* is an aliphatic $C_{1-4}$acyl, and $R_1$, $R_2$, $R_3$ and *n* are as defined above, a thiocarboxylic S-acid of the formula R—COSH, in which R is an alkyl group, is reacted, with a halogenated derivatice of the formula:

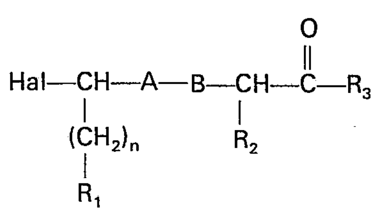

(V)

in which Hal is halogen, particularly bromine, for the preparation of derivatives of the formula (I), in which: *A*=CO, *B*=NH, *Y*=S, *X* is $C_{1-2}$ alkyl substituted with mercapto, aliphatic acylthio, or benzoylthio and $R_1$, $R_2$, $R_3$ and *n* are as defined above, a halogenated compound of the formula:

R—CO—S—$(CH_2)_m$—Hal

in which Hal is halogen such as bromine, R is an alkyl or phenyl group and *m* is 1 or 2, is reacted with a derivative having the formula:

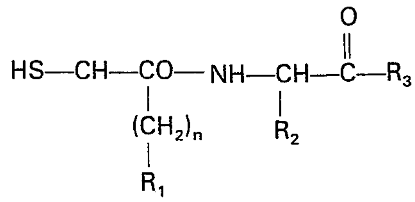

for the preparation of derivatives of the formula (I), in which: *A*=NH, *B*=CO, *Y*=NH, *X* is a hydrogen, an alkyl substituted with a carboxy or mercapto, or an alkoxycarbonyl group, and $R_1$, $R_2$, $R_3$ and *n* are as defined above, a peptidic condensation reaction is carried out between a N-protected diamine having the formula:

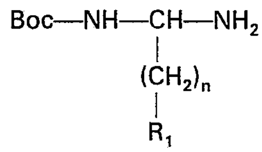
(VI)

and a malonic monoester or monoamides of the formula:

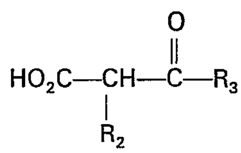

for the preparation of derivatives of the formula (I), in which: *A*=$CH_2$, *B*=S, *Y*=NH, *X* is a hydrogen, an alkyl substituted with a carboxy or mercapto, or an alkoxycarbonyl, and $R_1$, $R_2$, $R_3$ and *n* are as defined above, a condensation reaction is carried out between a N-protected tosylated aminoacid derivative having the formula:

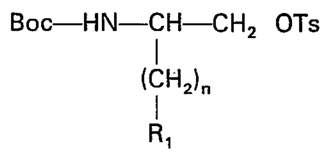
(VII)

and a thioglycolic acid ester or amide having the formula:

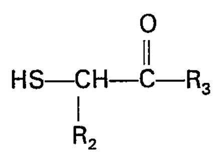
(VIII)

for the preparation of derivatives of the formula (I), in which: *A*=—$CH_2$, *B*=—NH—, *Y*=NH, *X* is an alkyl substituted with a carboxy or mercapto group, or an alkoxycarbonyl, and $R_1$, $R_2$, $R_3$ and *n* are as defined above, a condensation reaction is carried out between a N-protected tosylated aminoacid derivative having the formula:

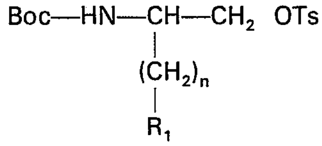

and an aminoacid ester or amide of the formula

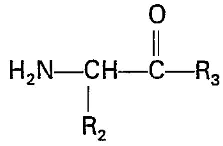

2. Process according to claim 1, characterized in that derivatives selected from the following are prepared:

N-[(R, S)-3-mercapto-2-benzyl-propionyl]-L-leucine;
N-[(R, S)-3-mercapto-2-benzyl-propionyl]-glycine;
N-[(R, S)-3-mercapto-2-benzyl-propionyl]-L-alanine;
O-benzyl, N-[(R, S)-3-mercapto-2-benzyl-propionyl]-L-serine;
N-[(R, S)-3-acetylthio-2-benzyl-propionyl]glycine benzyl ester;
N-[(R, S)-3-acetylthio-2-benzyl-propionyl]glycine p.fluorobenzyl ester;
N-[(R, S)-3-acetylthio-2-benzyl-propionyl]glycine 2,2,2-trifluoroethyl ester;
N-[(R, S)-3-acetylthio-2-benzyl-propionyl]glycine benzylamide;
N-[(R, S)-3-mercapto-2-benzyl-propionyl]glycine benzyl ester, and
N-[(R, S)-3-mercapto-2-benzyl-propionyl]-glycine 2,2,2-trifluoroethyl ester.

3. Process for the preparation of a medicament having particularly enkephalinase-inhibiting, antalgic, and hypotensive activities, characterized in that a compound according to claim 1 or 2 is put in a therapeutically administrable form.